# EUROPEAN PATENT APPLICATION

(11) **EP 4 764 335 A1**
(43) Date of publication of application: **24.06.2026**
(21) Application number: 25217633.4
(22) Date of filing: 21.11.2025
(51) Int. Cl.: F24F 8/10, F24F 8/108, F24F 11/30, F24F 3/167, F24F 11/00

(54) **INDOOR AIR PURIFICATION SYSTEM**

(30) Priority: 20.12.2024 TW 113149886
(71) Applicant: Microjet Technology Co., Ltd., Hsinchu (TW)
(72) Inventor: Mou, Hao-Jan, Hsinchu (TW); Wu, Chin-Chuan, Hsinchu (TW); Lin, Ching-Sung, Hsinchu (TW); Huang, Chi-Feng, Hsinchu (TW)
(74) Representative: Uexküll & Stolberg

(57) **Abstract**

An indoor field intelligent cleaning system includes a plurality of gas detection modules (1), an air pollution purification apparatus (2) and a networked cloud computing service device (3). The gas detection modules (1) continuously monitor the air quality data for automatically enabling the air pollution purification apparatus (2). The networked cloud computing service device (3) includes an AI smart calculation platform (35) with functions of AI smart control, intelligent energy management and malfunction diagnosis, the air condition of the indoor field (A) can rapidly be adjusted according to environmental real time variation, thereby optimizing systematic energy efficiency and maintaining best air quality. Accordingly, a continuously clean and healthy air environment of indoor field (A) can be created, and the impact of harmful pollutants in the air on human health also can be reduced.

## Description

### FIELD OF THE INVENTION

The present disclosure relates to air cleaning and intelligent control, and more particularly to a clean room intelligent processing system which relates to indoor field environment, and provides an indoor filed intelligent cleaning system for achieving the safety and comfort of the indoor field environment.

### BACKGROUND OF THE INVENTION

With air pollution becomes worse and pollution sources increases in modern urban environments, especially for babies whose immune systems are not yet fully developed, PM2.5 in particular may seriously cause otitis media, increase cancer risk and damage brain function. Thus, a clean air environment is important for human health. However, the conventional air cleaning system has difficulty in achieving an instant and precise air quality control, and is unable to automatically adjust according to the indoor environment for keeping the indoor air pollution at a level close to zero. Therefore, there is a need of an indoor filed intelligent cleaning system capable of instantly and intelligently monitoring and dynamically adjusting the indoor air quality.

### SUMMARY OF THE INVENTION

An object of the present disclosure is to provide an indoor filed intelligent cleaning system which combines efficient air purification technology with artificial intelligent control to achieve the automatic adjustment of indoor air quality, thereby creating a safe and clean breathing environment of indoor field.

In accordance with an aspect of the present disclosure, an indoor filed intelligent cleaning system includes a plurality of gas detection modules arranged in an indoor field and an outdoor field for detecting an air pollution and outputting an air quality data via Internet of Things (IoT) communication; at least one air pollution purification apparatus disposed in the indoor field and comprising at least one gas detection module, at least one air guiding fan, at least one filter element and at least one host driving controller, wherein the at least one gas detection module is electrically connected to the at least one host driving controller and receives a control instruction via Internet of Things (IoT) communication for enabling the at least one air guiding fan to circulate and purify the air pollution in the indoor field so as to reach a cleanness of cleanroom class with a level of air pollution close to zero; and at least one networked cloud computing service device receiving the air quality data outputted by the plurality of gas detection module via Internet of Things (IoT) communication, and performing real-time monitoring and analysis based on the collected air quality data. Wherein, the at least one networked cloud computing service device collects and monitors the air quality data in real time and intelligently issues a control instruction to the at least one gas detection module, so that the host driving controller is regulated to control an activation operation of the at least one air guiding fan, and dynamically adjust an operating frequency and an output air volume of the at least one air guiding fan so as to circulate and purify the air pollution in the indoor field, thereby reaching the cleanness of cleanroom class with the level of air pollution close to zero.

### BRIEF DESCRIPTION OF THE DRAWINGS

The above contents of the present disclosure will become more readily apparent to those ordinarily skilled in the art after reviewing the following detailed description and accompanying drawings, in which:
FIG. 1A is a schematic view illustrating an indoor filed intelligent cleaning system according to an embodiment of the present disclosure;
FIG. 1B is a schematic view illustrating the architecture of an air pollution purification apparatus according to an embodiment of the present disclosure;
FIG. 1C is a schematic view illustrating the implementation (1) of the indoor filed intelligent cleaning system according to an embodiment of the present disclosure;
FIG. 1D is a schematic view illustrating the implementation (2) of the indoor filed intelligent cleaning system according to another embodiment of the present disclosure;
FIG. 1E is a schematic view illustrating the implementation (3) of the indoor filed intelligent cleaning system according to another embodiment of the present disclosure;
FIG. 2A is a schematic diagram illustrating the architecture of a gas detection module of the indoor filed intelligent cleaning system according to an embodiment of the present disclosure;
FIG. 2B is a schematic view illustrating a gas exchange device of the air pollution purification apparatus according to an embodiment of the present disclosure;
FIG. 2C is a schematic view illustrating a purifier of the air pollution purification apparatus according to an embodiment of the present disclosure;
FIG. 2D is a schematic cross-sectional view illustrating the purifier of the air pollution purification apparatus shown in FIG. 2A and FIG. 2C;
FIG. 2E is a schematic view illustrating a fan filter unit (FFU) of the air pollution purification apparatus according to an embodiment of the present disclosure;
FIG. 2F is a schematic cross-sectional view illustrating a humidity controller of the air pollution purification apparatus shown in FIG. 2A;
FIG. 2G is a schematic diagram illustrating a process of comparing a pressure difference of carbon dioxide (CO₂) between indoor field and outdoor field so as to control a positive pressure air intake, wherein the process is performed by the gas exchange device through the networked cloud computing service device according to an embodiment of the present disclosure;
FIG. 2H is a schematic view illustrating the combination of filter elements of the air pollution purification apparatus according to an embodiment of the present disclosure;
FIG. 3A is a schematic perspective view illustrating a gas detection module according to an embodiment of the present disclosure;
FIG. 3B is a schematic perspective view illustrating the gas detection module according to the embodiment of the present disclosure from another view angle;
FIG. 4A is a schematic perspective view (1) illustrating a gas detection main part according to the embodiment of the present disclosure;
FIG. 4B is a schematic perspective view (2) illustrating the gas detection main part according to the embodiment of the present disclosure;
FIG. 4C is an exploded view illustrating the gas detection module according to the embodiment of the present disclosure;
FIG. 5A is a schematic perspective view (1) illustrating a base according to the embodiment of the present disclosure;
FIG. 5B is a schematic perspective view (2) illustrating the base according to the embodiment of the present disclosure;
FIG. 6 is a schematic view (3) illustrating the base according to the embodiment of the present disclosure;
FIG. 7A is a schematic exploded view illustrating a piezoelectric actuator and the base according to the embodiment of the present disclosure;
FIG. 7B is a schematic perspective view illustrating the combination of the piezoelectric actuator and the base according to the embodiment of the present disclosure;
FIG. 8A is a schematic exploded view (1) illustrating the piezoelectric actuator according to the embodiment of the present disclosure;
FIG. 8B is a schematic exploded view (2) illustrating the piezoelectric actuator according to the embodiment of the present disclosure;
FIG. 9A is a schematic cross-sectional view (1) illustrating an action of the piezoelectric actuator according to the embodiment of the present disclosure;
FIG. 9B is a schematic cross-sectional view (2) illustrating an action of the piezoelectric actuator according to the embodiment of the present disclosure;
FIG. 9C is a schematic cross-sectional view (3) illustrating an action of the piezoelectric actuator according to the embodiment of the present disclosure;
FIG. 10A is a schematic cross-sectional view (1) illustrating the gas detection main part according to the embodiment of the present disclosure;
FIG. 10B is a schematic cross-sectional view (2) illustrating the gas detection main part according to the embodiment of the present disclosure;
FIG. 10C is a schematic cross-sectional view (3) illustrating the gas detection main part according to the embodiment of the present disclosure;
FIG. 11 is a block diagram illustrating the architecture of a networked cloud computing service device according to the embodiment of the present disclosure; and
FIG. 12 is a table showing the cleanness of cleanroom CLASS 7~12 achieved by the indoor filed intelligent cleaning system of the present disclosure through detecting and purifying the air pollution to a level close to zero.

### DETAILED DESCRIPTION OF THE PREFERRED EMBODIMENT

The present disclosure will now be described more specifically with reference to the following embodiments. It is to be noted that the following descriptions of preferred embodiments of this disclosure are presented herein for purpose of illustration and description only. It is not intended to be exhaustive or to be limited to the precise form disclosed.

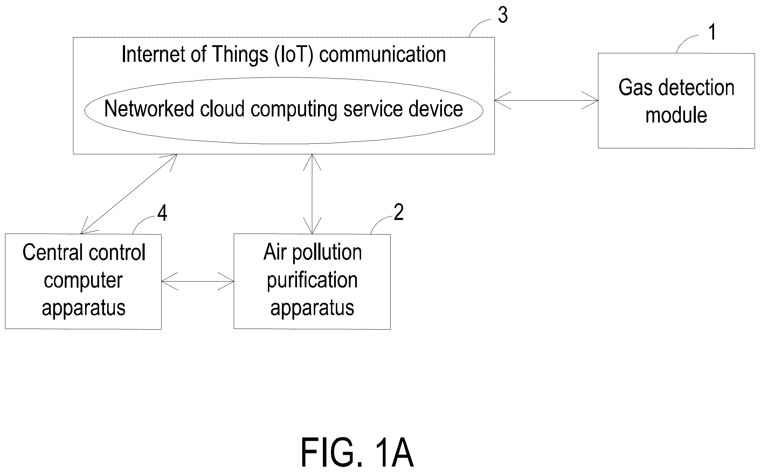

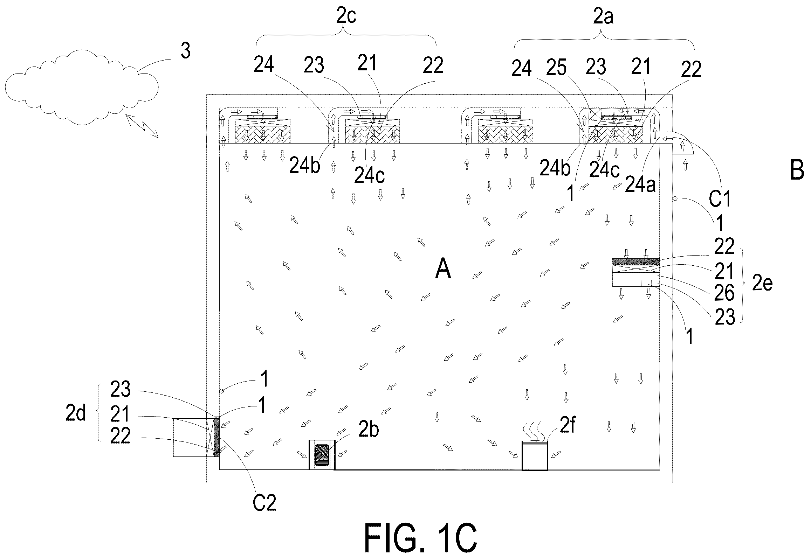

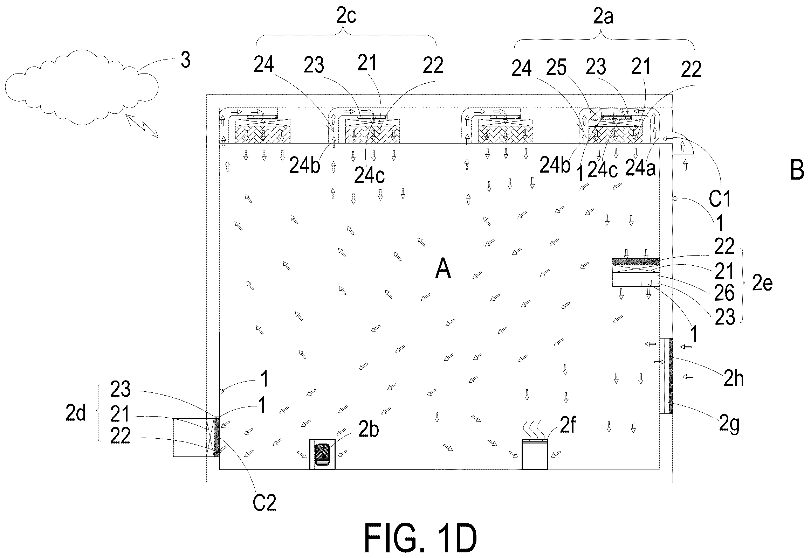

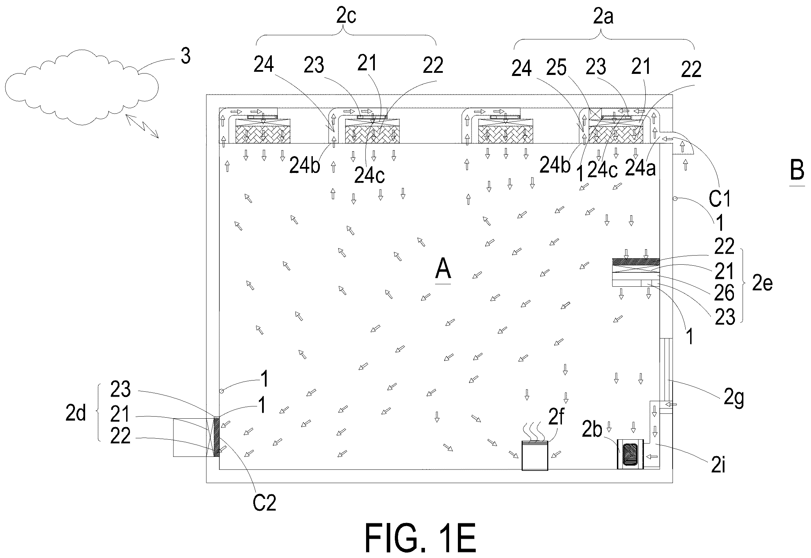

Please refer FIG. 1A. The present disclosure is related to an indoor filed intelligent cleaning system including a plurality of gas detection modules 1, at least one air pollution purification apparatus 2, at least one networked cloud computing service device 3 and at least one central control computer apparatus 4. The networked cloud computing service device 3 receives air quality data outputted by the gas detection modules 1 via Internet of Things (IoT) communication, and an AI smart calculation platform 35 of the networked cloud computing service device 3 analyzes the air quality data. Based on the analysis result, the networked cloud computing service device 3 intelligently issues a control instruction for automatically adjusting the operation mode of the air pollution purification apparatus 2 so as to circulate and purify the air pollution in the indoor field to a level close to zero, thereby reach a cleanness of cleanroom class for the indoor field A (as shown in FIG. 1C, FIG. 1D and FIG. 1E).

The above-mentioned gas detection modules 1 are installed in the indoor filed A and the outdoor field B for detecting the air quality data, such as particle matters (PM1, PM2.5, PM10), the concentration of carbon dioxide (CO₂), temperature and humidity. As shown in FIG. 1C, the plurality of gas detection modules 1 mentioned above are arranged in an indoor field A and an outdoor field B for detecting the air pollution and outputting the air quality data via Iot communication. Notably, the air quality data includes particle matters (PM1, PM2.5, PM10), the concentration of carbon dioxide (CO₂), temperature, humidity etc.

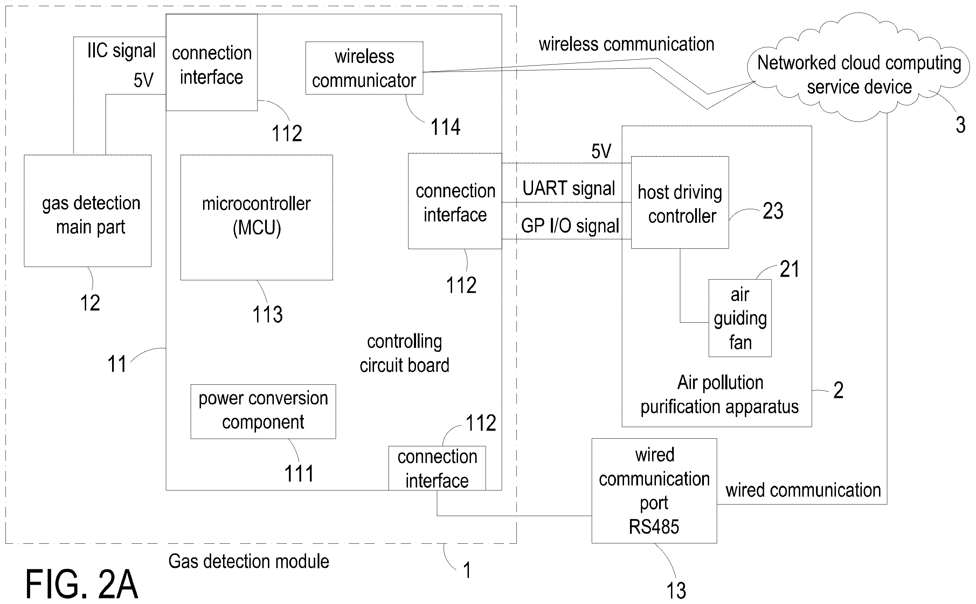

As shown in FIG. 1C and FIG. 2A, the air pollution purification apparatuses 2 mentioned above includes a gas exchange device 2a, a purifier 2b, a fan filter unit (FFU) 2c, an exhaust fan 2d, a heating and cooling air conditioner 2e, and a humidity controller 2f and is disposed in the indoor field A in a build-in manner or a plug-in manner. The gas exchange device 2a is implemented to achieve the ventilation of indoor field A and provide a positive pressure air intake for preventing air pollution from entering indoor field A. The purifier 2b, the fan filter unit (FFU) 2c and the exhaust fan 2d are provided for purifying the air pollution in the indoor field A to a level close to zero so as to reach a cleanness of cleanroom class. The heating and cooling air conditioner 2e and the humidity controller 2f are implemented to adjust the temperature and the humidity of indoor field A. Moreover, each air pollution purification apparatus 2 includes at least one gas detection module 1, at least one air guiding fan 21, at least one filter element 22 and at least one host driving controller 23 disposed therein. The gas detection module 1 is electrically connected to the host driving controller 23, receives the control instruction via IoT communication and provides the control instruction to the host driving controller 23 for enabling the air guiding fan 21, so that, according to the control instruction from the networked cloud computing service device 3, the gas detection module 1 is capable of automatically performing operations of air filtration, air exchanging, temperature and humidity adjustment and sterilization, thereby circulating and purifying the air pollution in the indoor field A to reach a cleanness of classroom class.

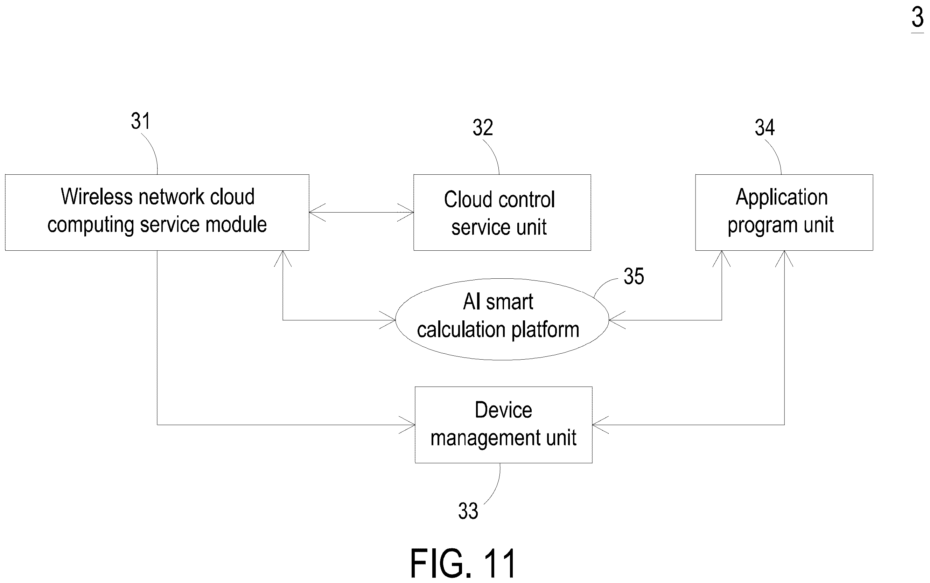

Please refer to FIG. 11. In the embodiment, the networked cloud computing service device 3 mentioned above includes a wireless network cloud computing service module 31, a cloud control service unit 32, a device management unit 33, an application program unit 34 and an AI smart calculation platform 35. The wireless network cloud computing service module 31 receives air quality data of the outdoor field B and the indoor field A, receives communication information of the air pollution purification apparatus(es) 2, and issues the control instruction. Moreover, the wireless network cloud computing service module 31 receives the air quality data of the indoor field A and the outdoor field B and transmits thereof to the cloud control service unit 32 to store and form an big data database of air pollution data, and performs an intelligence computing and comparison based on the big data database of air pollution data for issuing the control instruction to the wireless network cloud computing service module 31 and then to the air pollution purification apparatus(es) 2 through the wireless network cloud computing service module 31 for enabling the air pollution purification apparatus(es) 2. The device management unit 33 receives the communication information of the air pollution purification apparatus(es) 2 through the wireless network cloud computing service module 31 for managing the user login and device binding, and also provides the management information to the application program unit 34 for system control and management, wherein the management information may include the maintenance and management, and the automatic abnormal point detection, analysis, treatment and improvement of the air pollution purification apparatuses(es) 2, whether the inspection and measurement is controlled to reach the cleanness requirement of cleanroom class, the customer demand feedback, and the correction mechanism for software and hardware technology improvement. The application program unit 34 can also display and inform the air quality information obtained from the cloud control service unit 32, so the user can know the real-time status of air pollution removal through the mobile phone or the communication device. Moreover, the user can control the operation of the indoor filed intelligent cleaning system through the application program unit 34 of the mobile phone or the communication device. The AI smart calculation platform 35 receives the air quality data from the gas detection module 1 through the IoT communication, analyzes thereof, and generates the control instruction based on the analysis result, so as to achieve the automatic control and optimization of the air pollution purification apparatus(es) 2, thereby automatically adjusting the operation mode of the air pollution purification apparatus(es) 2.

The AI smart calculation platform 35 includes functions of AI smart control, intelligent energy management and automatic malfunction diagnosis. The AI smart control performs calculation based on the air quality data and automatically adjusts parameters of air volume and purification mode through the preset calculation, so as to precisely control the operation of the air pollution purification apparatus(es) 2 according to the air quality data (such as PM2.5) detected in the indoor field A in real time, thereby optimizing the systematic energy efficiency and maintaining a best air quality. The intelligent energy management dynamically adjusts the usage of energy according to the environmental condition of the indoor field A and the operation of the air pollution purification apparatus(es) 2 so as to meet the requirements for cleanroom, wherein the temperature and humidity of the environment condition of the indoor field A is precisely controlled within a range suitable for human health, and the power consumption is automatically reduced when the air pollution purification apparatus(es) 2 is/are idle so as to effectively save energy and minimize energy consumption. The automatic malfunction diagnosis automatically generates the report and notifies the user when the air pollution purification apparatus(es) 2 operate(s) abnormally, for promptly alarming and providing maintenance suggestions, such that the apparatus(es) can be monitored, maintained and repaired in time, and potential failures also can be predicted, especially for the automatic cleaning of the filter element 22 and the gas guiding channel 24, which reduces the requirements of daily maintenances and helps in maintaining the long term and efficient operation of the apparatus(es).

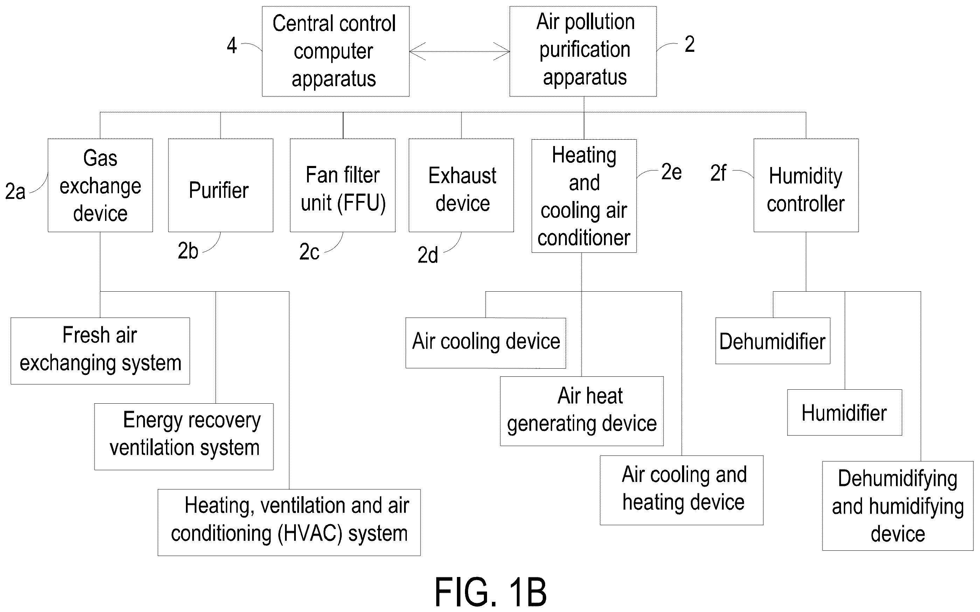

As shown in FIG. 1A and FIG. 1B, the central control computer apparatus 4 receives the control instruction from the networked cloud computing service device 3 via Internet of Things (IoT) communication, and transmits thereof to the gas detection module 1 of each air pollution purification apparatus 2, so as to enable the air guiding fan 21. Alternatively, the central control computer apparatus 4 includes a function of edge computing for calculating and analyzing the air quality data received from the gas detection module 1 in each air pollution purification apparatus 2, generating the control instruction based on the analysis result and directly issuing the control instruction to the gas detection module 1 of each air pollution purification apparatus 2 via Internet of Things (IoT) communication so as to enable the air guiding fan 21, thereby achieving the automatic control and the optimization of the air pollution purification apparatus(es) 2.

Notably, the Internet of Things (IoT) communication mentioned above refers to the collective network which connects various devices and the mutual communication technology between devices and between device and the cloud. The IoT communication can provide wired communication for the networked cloud computing service device 3 to communicate in a wired manner. The IoT communication also can provide wireless communication for the networked cloud computing service device 3 to communicate wirelessly. Preferably but not exclusively, the wireless communication is one selected from the group consisting of a Wi-Fi communication, a Bluetooth communication, a radio frequency identification communication and a near field communication.

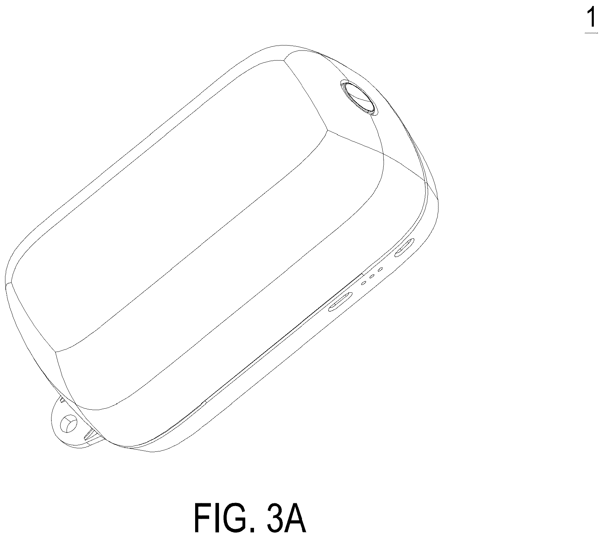

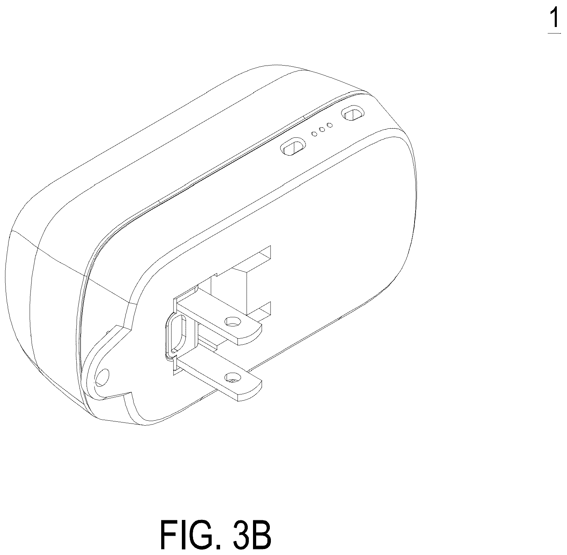

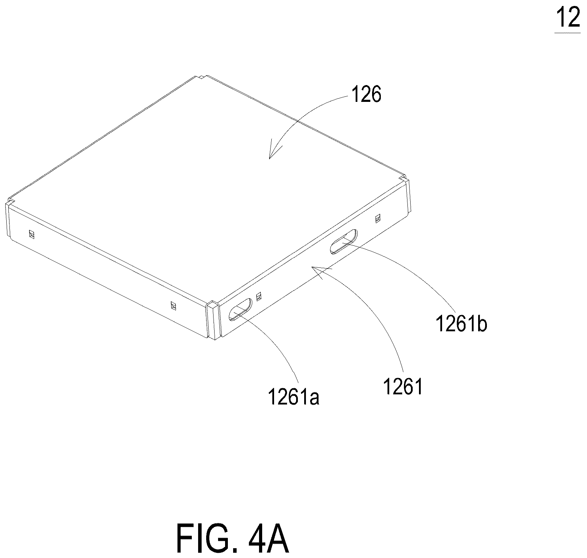

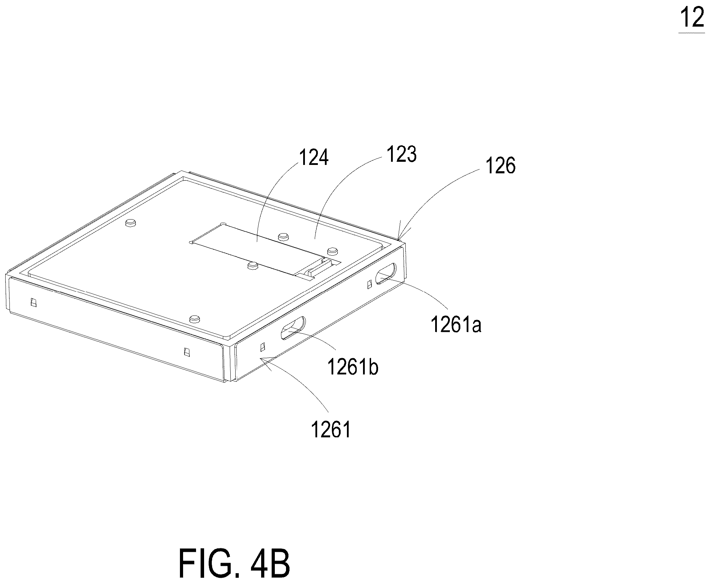

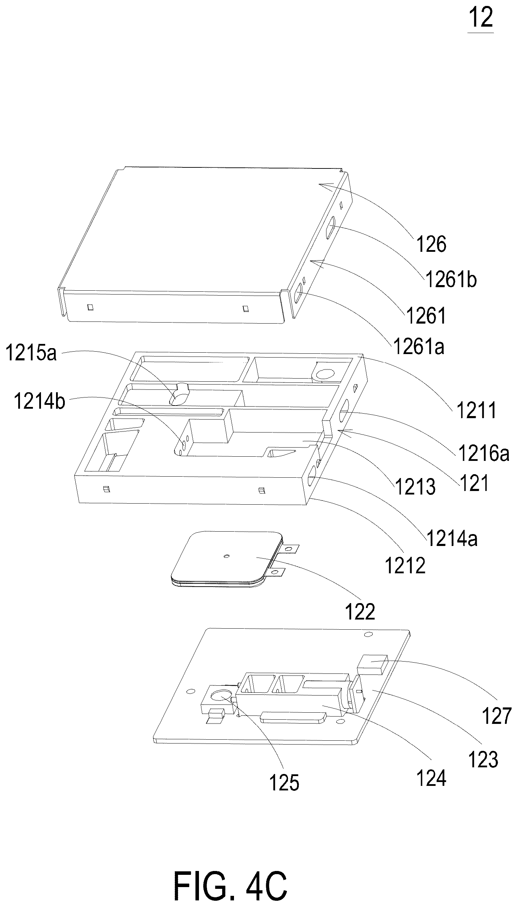

Notably, as shown in FIG. 3A and FIG. 3B, the gas detection module 1 is configured with an external power terminal, and the external power terminal can be directly inserted into the power interface in the indoor field A for enabling the detection of the air quality data of air pollution, concentration of carbon dioxide (CO₂), temperature and humidity. Alternatively, as shown in FIG. 4A, the gas detection module 1 is configured without external power supply terminals and is directly disposed on and electrically connected to the air pollution purification apparatus 2 for receiving the control instruction for controlling the power supply of the air pollution purification apparatus 2 and thus controlling the enablement of the air guiding fan 21.

Please refer to FIG. 2A. In the embodiment, the gas detection module 1 includes a controlling circuit board 11 and a gas detection main part 12. The gas detection main part 12 detects the humidity, the temperature and the air pollution to generate the air quality data. The controlling circuit board 11 collects, calculates, analyzes and outputs the air quality data to form a serial communication (IIC) signal for input, and the networked cloud computing service device 3 receives and analyzes the air quality data in real time to output a Universal Asynchronous Transceiver and Transceiver (UART) signal and a General Purpose Input and Output (GP I/O) signal for the host driving controller 23.

In the embodiment, the controlling circuit board 11 includes a power conversion component 111, at least one connection interfaces 112, a microcontroller (MCU) 113 and a wireless communicator 114. The power conversion component 111 provides DC voltage division modulation to output a required DC voltage. The required DC voltage is transmitted through at least one connection interface 112 to the gas detection main part 12 for actuation operation and to the host driving controller 23 for actuation operation. The microcontroller (MCU) 113 is connected to the gas detection main part 12 through the at least one connection interface 112 to form the serial communication (IIC) signal for input, so that the air quality data is calculated and analyzed, and connected through the at least one connection interface 112 to output the Universal Asynchronous Transceiver and Transceiver (UART) signal and the General Purpose Input and Output (GP I/O) signal for regulation of the host driving controller 23. The wireless communicator 114 receives the air quality data and transmits the air quality data to the networked cloud computing service device 3 through external wireless communication. The networked cloud computing service device 3 collects, analyzes and monitors the air quality data in real time and intelligently selects a control command, and the control command is received through the wireless communicator 114 and transmitted to the microcontroller (MCU) 113 to output the Universal Asynchronous Transceiver and Transceiver (UART) signal and the General Purpose Input and Output (GP I/O) signal for regulation of the host driving controller 23, so that the host driving controller 23 is regulated to control the activation operation of the at least one air guiding fan 21 and dynamically adjust the operating frequency and the output air volume of the at least one air guiding fan 21.

In an embodiment, the gas detection module 1 inside the air pollution purification apparatus 2 further includes a wired communication port 13. The wired communication port 13 is electrically connected to the controlling circuit board 11 through a connection interface 112 for external connection to a wired communication transmission. The air quality data is received and transmitted to the networked cloud computing service device 3 through external wireless communication, the networked cloud computing service device 3 collects, analyzes and monitors the air quality data in real time and intelligently selects a control command, and the control command is received through the wired communication port 13 and transmitted to the microcontroller (MCU) 113 to output the Universal Asynchronous Transceiver and Transceiver (UART) signal and the General Purpose Input and Output (GP I/O) signal for regulation of the host driving controller 23, so that the host driving controller 23 is regulated to control the activation operation of the at least one air guiding fan 21, and dynamically adjust the operating frequency and the output air volume of the at least one air guiding fan 2. Notably, in the embodiment, the wired communication port 13 is an RS485 port that communicates with the networked cloud computing service device 3 through a wired line connection.

Notably, in the embodiment, the air pollution includes at least one selected from the group consisting of particulate matter, carbon monoxide, carbon dioxide, ozone, sulfur dioxide, nitrogen dioxide, lead, total volatile organic compounds (TVOC), formaldehyde, bacteria, fungi, virus and a combination thereof.

Followings are embodiments of the air pollution purification apparatus 2 disposed in the indoor field A. As shown in FIG. 1C, the air pollution purification apparatuses 2 are disposed in the indoor field A, and the indoor field A includes at least one gas introducing opening C1 and at least one gas discharging opening C2.

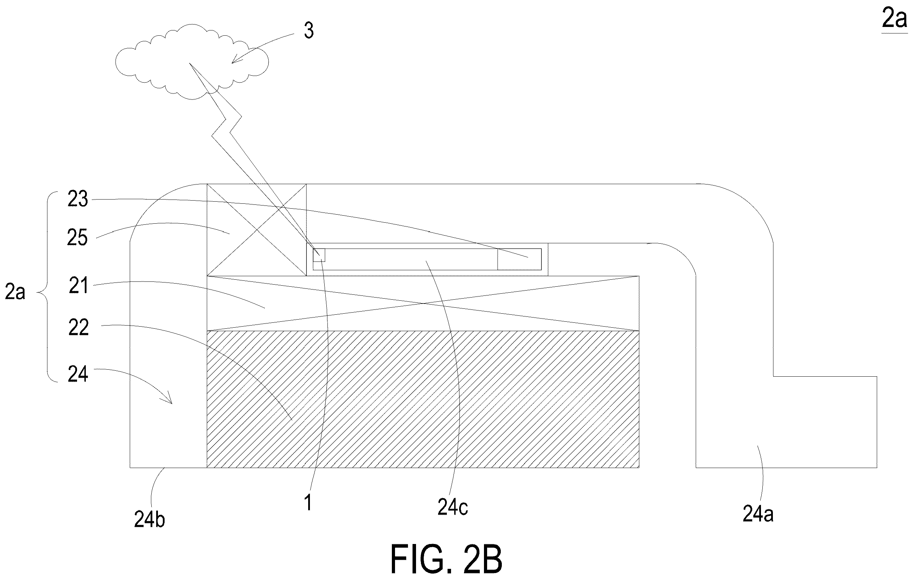

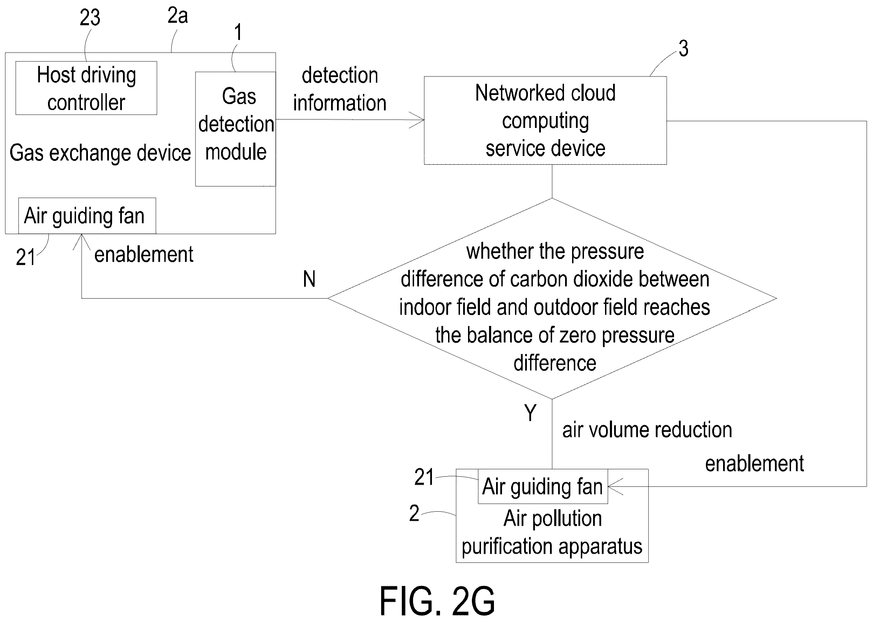

As shown in FIG. 1C and FIG. 2B, the gas exchange device 2a includes a gas guiding channel 24. The gas guiding channel 24 includes a gas introducing port 24a corresponding to the gas introducing opening C1 of the indoor field A, a circulation returning port 24b communicated with the indoor field A, and a gas filtering channel 24c communicated with the indoor field A. A gas exchanger 25 is disposed near the circulation returning port 24b, and the gas filtering channel 24c has the air guiding fan 21 and the filter element 22 disposed therein. In the embodiment, the networked cloud computing service device 3 intelligently computes to compare the carbon dioxide (CO₂) pressure detection information of the indoor field A and the outdoor field B, and the safety value of the carbon dioxide (CO₂) pressure detection information of the indoor field A must be maintained at 400~600 ppm. As shown in FIG. 2G, when the networked cloud computing service device 3 receives the detection information of the gas exchange device 2a via Internet of Things (IoT) communication, the pressures of carbon dioxide (CO₂) in the indoor field A and in the outdoor field B are compared to check whether the balance of zero pressure difference is reached (namely, the carbon dioxide (CO₂) pressure detection information of the indoor field A and the outdoor field B are the same to achieve the balance). When the balance of zero pressure difference is not reached, the control instruction is selectively issued to the gas detection module 1 of the gas exchange device 2a for controlling the host driving controller 23 to enable the air guiding fan 21. Accordingly, the gas in the outdoor field B is introduced into the gas filtering channel 24c through the gas introducing opening C1, is filtered through passing through the filter element 22, and enters the indoor field A, and at the same time, the gas in the indoor field A enters the gas filtering channel 24c again through the circulation returning port 24b for circulation and filtration, thereby achieving temperature adjustment and gas exchange. The gas exchange is implemented to reach a level of carbon dioxide pressure difference between the indoor field A and the outdoor field B close to zero. Notably, when the gas exchange device 2a is enabled to perform the gas exchange, a positive pressure higher than 0 Pa must be maintained in the indoor field A, so that the air pollution in the outdoor field B does not enter the indoor field A. In the embodiment, the gas detection module 1 inside the air pollution purification apparatus 2 continuously receives the control instruction from the networked cloud computing service device 3 for controlling the host driving controller 23 to enable the air guiding fan 21, and continuously circulates and purifies the air pollution in the indoor field A to reach the cleanness of cleanroom class with the level of air pollution close to zero and adjust the temperature and humidity. When the networked cloud computing service device 3 performs comparison and confirms that the pressure difference of carbon dioxide (CO₂) between the indoor field A and the outdoor field B reaches the balance of zero pressure difference, the networked cloud computing service device 3 issues the control instruction to the gas detection module 1 inside the air pollution purification apparatus 2 for controlling the host driving controller 23 to reduce the air volume of the air guiding fan 21, so as to save energy consumption and effectively reduce the generation of airflow noises, thereby achieving a real-time air pollution detection and purification for reaching the cleanness of cleanroom class with the level of air pollution close to zero. Notably, as shown in FIG. 1B, the gas exchange device 2a is a fresh air exchanging system, or the gas exchange device 2a is an energy recovery ventilation system, or the gas exchange device 2a is a heating, ventilation and air conditioning (HVAC) system, but not limited thereto.

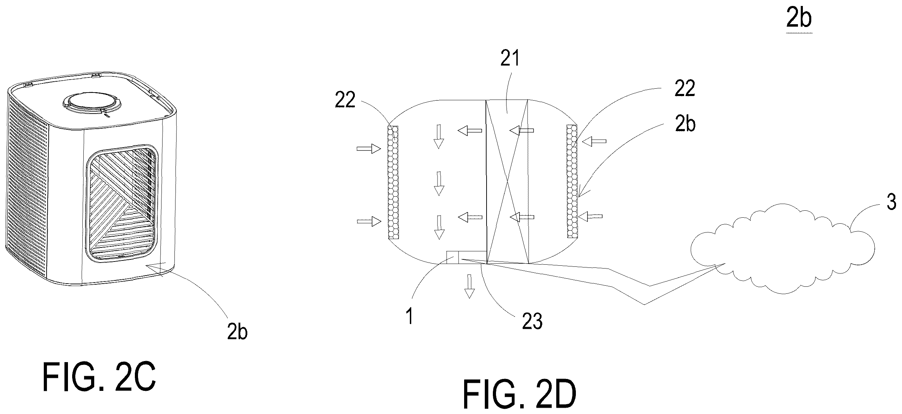

As shown in FIG. 1C, FIG. 2C and FIG. 2D, the purifier 2b is disposed in the indoor field A in a plug-in manner, and the networked cloud computing service device 3 issues the control instruction via IoT communication to the gas detection module 1 inside the purifier 2b for controlling the host driving controller 23 to enable the air guiding fan 21, so that the air pollution in the indoor field A is guided to pass through the filter element 22 for filtration and purification, and then, the purified air is introduced into the indoor field A again. Therefore, the air pollution in the indoor field A may be guided to pass through the filter element 22 multiple times to execute the purification procedure for reaching the cleanness of cleanroom class with the level of air pollution close to zero.

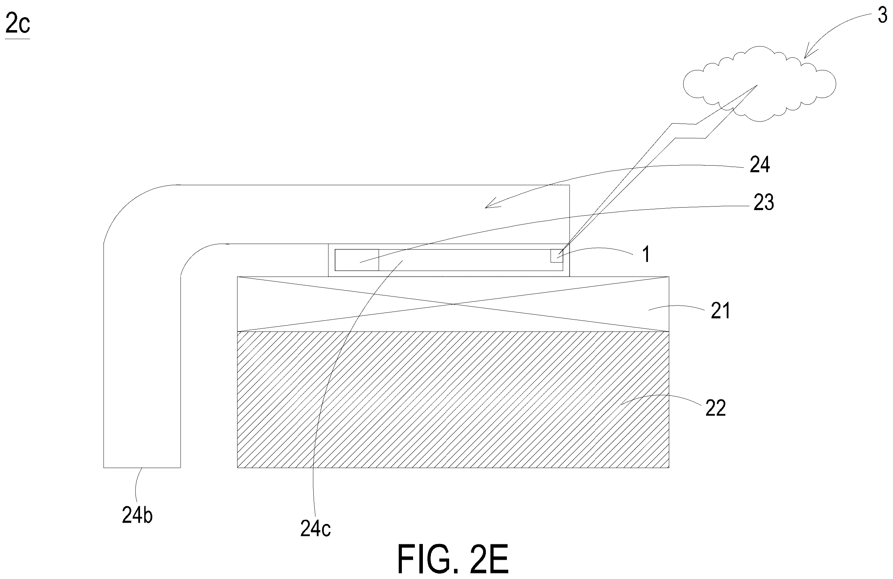

As shown in FIG. 1C and FIG. 2E, the fan filter unit (FFU) 2c is disposed in the indoor field A in a build-in manner. The fan filter unit (FFU) 2c includes a guiding channel 24. The gas guiding channel 24 includes a circulation returning port 24b communicated with the indoor field A, and a gas filtering channel 24c communicated with the indoor field A, wherein the gas filtering channel 24c has the air guiding fan 21 and the filter element 22 disposed therein. In that embodiment, the networked cloud computing service device 3 issues the control instruction via IoT communication to the gas detection module 1 inside the fan filter unit (FFU) 2c for controlling the host driving controller 23 to enable the air guiding fan 21, so that the air pollution in the indoor field A is guided to enter the gas filtering channel 24c through the circulation returning port 24b and pass through the filter element 22 for filtration and purification, and then, the purified air is introduced into the indoor field A again. Therefore, the air pollution in the indoor filed A is guided to enter the guiding channel 24 multiple times for effectively inhibiting the gas backflow effect during circulation and filtration, thereby achieving the purification procedure for reaching the cleanness of cleanroom class with the level of air pollution close to zero.

As shown in FIG. 1C, the exhaust fan 2d is disposed in the indoor field A in a build-in manner, and is corresponding to the gas discharging opening C2 for exhausting the gas to the outdoor field B. In the embodiment, the networked cloud computing service device 3 issues the control instruction via IoT communication to the gas detection module 1 inside the exhaust fan 2d for controlling the host driving controller 23 to enable the air guiding fan 21, so that the air pollution in the indoor field A is guided by the air guiding fan 21 to pass through the filter element 22 for filtration and purification and exhaust to the outdoor field B, thereby achieving the purification procedure for reaching the cleanness of cleanroom class with the level of air pollution close to zero.

As shown in FIG. 1C, the heating and cooling air conditioner 2e is disposed in the indoor field A, and the heating and cooling air conditioner 2e includes a temperature regulator 26. In the embodiment, the networked cloud computing service device 3 issues the control instruction via IoT communication to the gas detection module 1 inside the heating and cooling air conditioner 2e for controlling the host driving controller 23 to enable the air guiding fan 21, so that the gas is guided to pass through the temperature regulator 26 for adjusting temperature and humidity of gas in the indoor field A. Also, the gas detection module 1 outputs the temperature and humidity information of gas of the indoor field A. Notably, the heating and cooling air conditioner 2e is set to adjust the temperature of the indoor field A at 25°C±3°C and the humidity of the indoor field A at 50%±10%. Notably, as shown in FIG. 1B, the heating and cooling air conditioner 2e is an air cooling device, or the heating and cooling air conditioner 2e is an air heat generating device, or the heating and cooling air conditioner 2e is an air cooling and heating device, but not limited thereto.

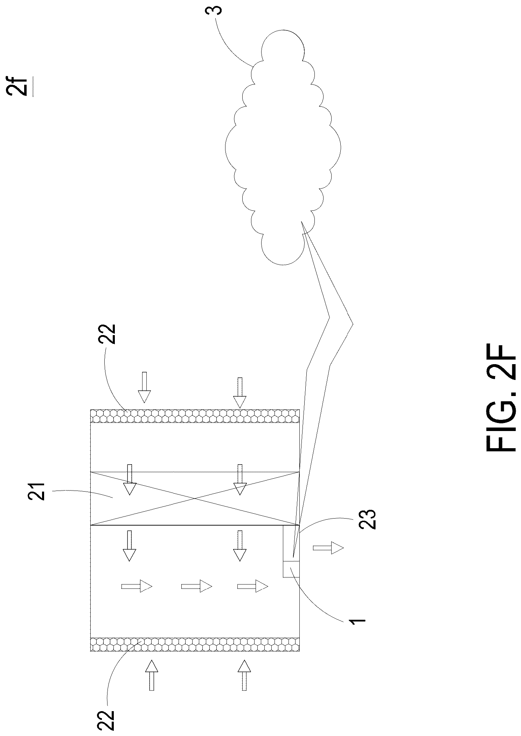

As shown in FIG. 1C and FIG. 2F, the humidity controller 2f is disposed in the indoor field A in a plug-in manner, and the networked cloud computing service device 3 issues the control instruction via IoT communication to the gas detection module 1 inside the humidity controller 2f for controlling the host driving controller 23 to enable the air guiding fan 21, so that the air pollution in the indoor field A is guided to pass through the filter element 22 for filtration and purification, thereby achieving the purification procedure for reaching the cleanness of cleanroom class with the level of air pollution close to zero, and adjusting the temperature and humidity of gas in the indoor field A. Notably, the humidity controller 2f is set to have a safety value of temperature at 25°C±3°C, and a safety value of humidity at 50%±10%. Notably, as shown in FIG. 1B, the humidity controller 2f is a dehumidifier, or the humidity controller 2f is a humidifier, or the humidity controller 2f is a dehumidifying and humidifying device, but not limited thereto.

In the implementation of the indoor filed intelligent cleaning system of the present disclosure, the air pollution purification apparatus 2 is disposed in the indoor field A. As shown in FIG. 1D, the indoor field intelligent cleaning system further comprises at least one airtight window 2g for preventing the air pollution. In the embodiment, the airtight window 2g includes a filter window 2h for filtering the air pollution. In some embodiments, the filter window 2h is an electrostatic filter to prevent the air pollution of the outdoor field B from entering the indoor filed A. In other embodiments, the filter window 2h is a filter woven from nanofibers, but not limited thereto. When the at least one gas detection module 1 detects that the concentration of carbon dioxide (CO₂) of the air quality data of the indoor field is too high, the airtight window 2g can be opened to allow gas exchange between the indoor field A and the outdoor field B, wherein the air pollution from the outdoor field B can be filtered through the filter window 2h to prevent it from entering the indoor field A. Meanwhile, the at least one air pollution purification apparatus 2 arranged in the indoor field A continuously monitors and automatically adjusts to purify the air pollution in the indoor field A close to zero, thereby effectively saving energy, minimizing energy consumption and maintaining a best air quality of the indoor field A. As shown in FIG. 1E, the indoor field intelligent cleaning system further comprises at least one airtight window 2g for preventing the air pollution. In this embodiment, the airtight window 2g includes a gas exchange channel 2i, wherein the gas exchange channel 2i is fluidly communicated with the outdoor field B and an inlet of the at least one air pollution purification apparatus 2 (as the inlet of the purifier 2b shown in FIG. 1E), so that the air in the outdoor field B is introduced by the at least one air guiding fan 21, enters the indoor field 2A through the gas exchange channel 2i and the at least one filter element 22 for gas exchange, so as to maintain the balance of carbon dioxide (CO₂) of the air quality data between the indoor field A and the outdoor field B.

According to descriptions above, the present disclosure provides an indoor filed intelligent cleaning system, wherein through the gas detection module 1 continuously monitoring the air quality data, including temperature, humidity, concentration of carbon dioxide, PM2.5, for automatically enabling the air pollution purification apparatus 2, and simultaneously, through cooperating with the AI smart calculation platform 35 of the networked cloud computing service device 3 with the functions of AI smart control, intelligent energy management and malfunction diagnosis, the air condition of the indoor field A can rapidly be adjusted according to the real time variation of environment, thereby optimizing the systematic energy efficiency and maintaining a best air quality. Accordingly, a continuously clean and healthy air environment of the indoor field A can be created, and the impact of harmful pollutants in the air on human health also can be reduced.

In the implementation of the indoor filed intelligent cleaning system of the present disclosure, through the gas detection module 1 continuously monitoring the air quality data, including temperature, humidity, concentration of carbon dioxide, PM2.5, for automatically enabling the air pollution purification apparatus 2, and simultaneously, through cooperating with the AI smart calculation platform 35 of the networked cloud computing service device 3 with the functions of AI smart control, intelligent energy management and malfunction diagnosis, the air condition of the clean room can rapidly be adjusted according to the real time variation of environment, thereby optimizing the systematic energy efficiency and maintaining a best air quality, and also, achieving a real time air pollution detection and purification to reach the cleanness of cleanroom CLASS 7~12.

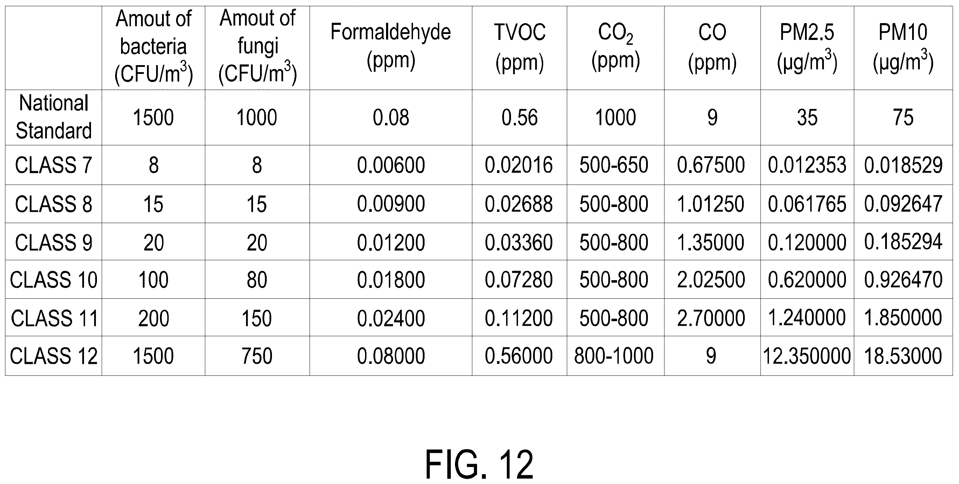

Please refer to FIG. 12, which shows the cleanness of cleanroom CLASS 7~12. Through the indoor filed intelligent cleaning system of the present disclosure, the air pollution in the indoor field A can be purified in real time to reach the cleanness of cleanroom class better than the national standards, in which the cleanness is less than or equal to 1500 CFU/m³ of the amount of bacteria, less than or equal to 1000 CFU/m³ of the amount of fungi, the average value per hour less than or equal to 0.08 ppm of the formaldehyde content, the average value per hour less than or equal to 0.56 ppm of the total volatile organic compounds (TVOC) content, the average value per 8 hours less than or equal to 1000 ppm of the carbon dioxide (CO₂) content, the average value per 8 hours less than or equal to 9 ppm of the carbon monoxide (CO) content, the average value less than or equal to 35 µg/m³ of PM2.5, and the average value less than or equal to 75 µg/m³ of PM10. Therefore, the best air quality can be maintained to create a continuously clan and healthy air environment for babies, and reduce the impact of harmful pollutants in the air on babies. The standard cleanness of cleanroom CLASS 7~12 are as follows.

The cleanness of CLASS 7 is less than or equal to 8 CFU/m³ of the amount of bacteria, less than or equal to 8 CFU/m³ of the amount of fungi, the average value per hour less than or equal to 0.00600 ppm of the formaldehyde content, the average value per hour less than or equal to 0.02016 ppm of the total volatile organic compounds (TVOC) content, the average value per 8 hours less than or equal to 500~650 ppm of the carbon dioxide (CO₂) content, the average value per 8 hours less than or equal to 0.67500 ppm of the carbon monoxide (CO) content, the average value less than or equal to 0.012353 µg/m³ of PM2.5, and the average value less than or equal to 0.018529 µg/m³ of PM10.

The cleanness of CLASS 8 is less than or equal to 15 CFU/m³ of the amount of bacteria, less than or equal to 15 CFU/m³ of the amount of fungi, the average value per hour less than or equal to 0.00900 ppm of the formaldehyde content, the average value per hour less than or equal to 0.02688 ppm of the total volatile organic compounds (TVOC) content, the average value per 8 hours less than or equal to 500~800 ppm of the carbon dioxide (CO₂) content, the average value per 8 hours less than or equal to 1.01250 ppm of the carbon monoxide (CO) content, the average value less than or equal to 0.061765 µg/m³ of PM2.5, and the average value less than or equal to 0.092647 µg/m³ of PM10.

The cleanness of CLASS 9 is less than or equal to 20 CFU/m³ of the amount of bacteria, less than or equal to 20 CFU/m³ of the amount of fungi, the average value per hour less than or equal to 0.01200 ppm of the formaldehyde content, the average value per hour less than or equal to 0.03360 ppm of the total volatile organic compounds (TVOC) content, the average value per 8 hours less than or equal to 500~800 ppm of the carbon dioxide (CO₂) content, the average value per 8 hours less than or equal to 1.35000 ppm of the carbon monoxide (CO) content, the average value less than or equal to 0.120000 µg/m³ of PM2.5, and the average value less than or equal to 0.185294 µg/m³ of PM10.

The cleanness of CLASS 10 is less than or equal to 100 CFU/m³ of the amount of bacteria, less than or equal to 80 CFU/m³ of the amount of fungi, the average value per hour less than or equal to 0.01800 ppm of the formaldehyde content, the average value per hour less than or equal to 0.07280 ppm of the total volatile organic compounds (TVOC) content, the average value per 8 hours less than or equal to 500~800 ppm of the carbon dioxide (CO₂) content, the average value per 8 hours less than or equal to 2.02500 ppm of the carbon monoxide (CO) content, the average value less than or equal to 0.62000 µg/m³ of PM2.5, and the average value less than or equal to 0.926470 µg/m³ of PM10.

The cleanness of CLASS 11 is less than or equal to 200 CFU/m³ of the amount of bacteria, less than or equal to 150 CFU/m³ of the amount of fungi, the average value per hour less than or equal to 0.02400 ppm of the formaldehyde content, the average value per hour less than or equal to 0.11200 ppm of the total volatile organic compounds (TVOC) content, the average value per 8 hours less than or equal to 500~800 ppm of the carbon dioxide (CO₂) content, the average value per 8 hours less than or equal to 2.70000 ppm of the carbon monoxide (CO) content, the average value less than or equal to 1.240000 µg/m³ of PM2.5, and the average value less than or equal to 1.850000 µg/m³ of PM10.

The cleanness of CLASS 12 is less than or equal to 1500 CFU/m³ of the amount of bacteria, less than or equal to 750 CFU/m³ of the amount of fungi, the average value per hour less than or equal to 0.08000 ppm of the formaldehyde content, the average value per hour less than or equal to 0.56000 ppm of the total volatile organic compounds (TVOC) content, the average value per 8 hours less than or equal to 800~1000 ppm of the carbon dioxide (CO₂) content, the average value per 8 hours less than or equal to 9 ppm of the carbon monoxide (CO) content, the average value less than or equal to 12.350000 µg/m³ of PM2.5, and the average value less than or equal to 18.53000 µg/m³ of PM10.

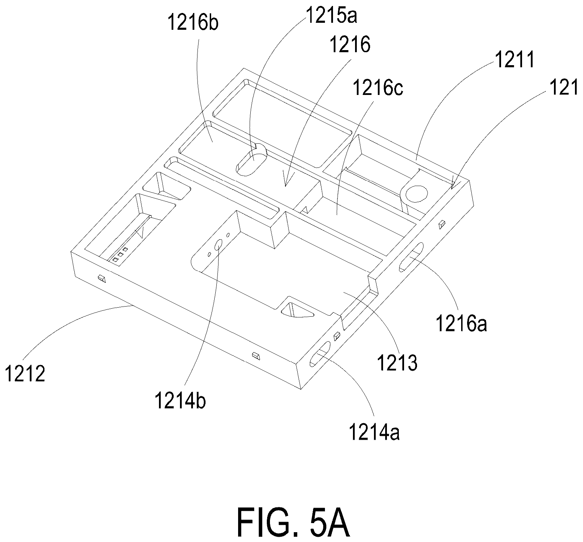

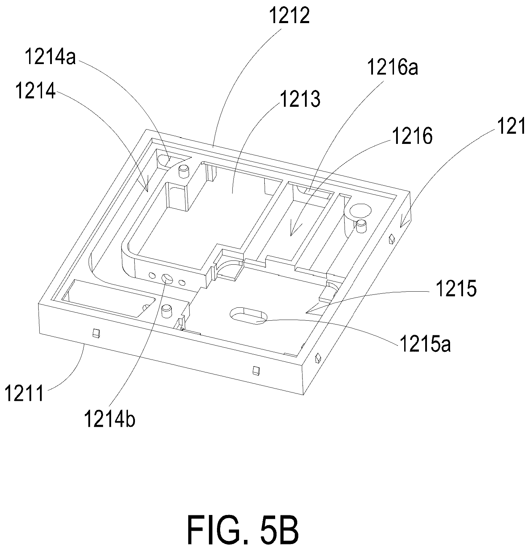

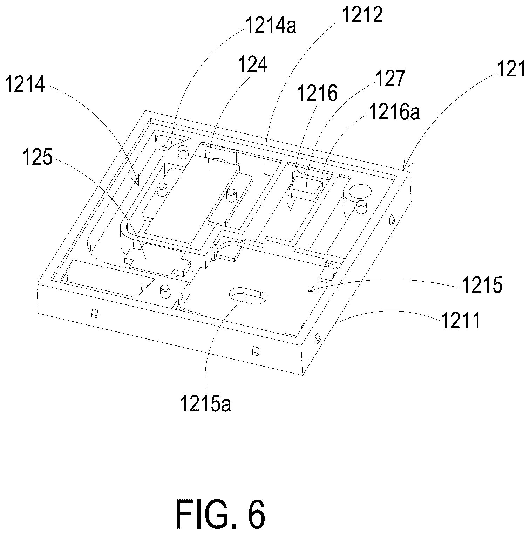

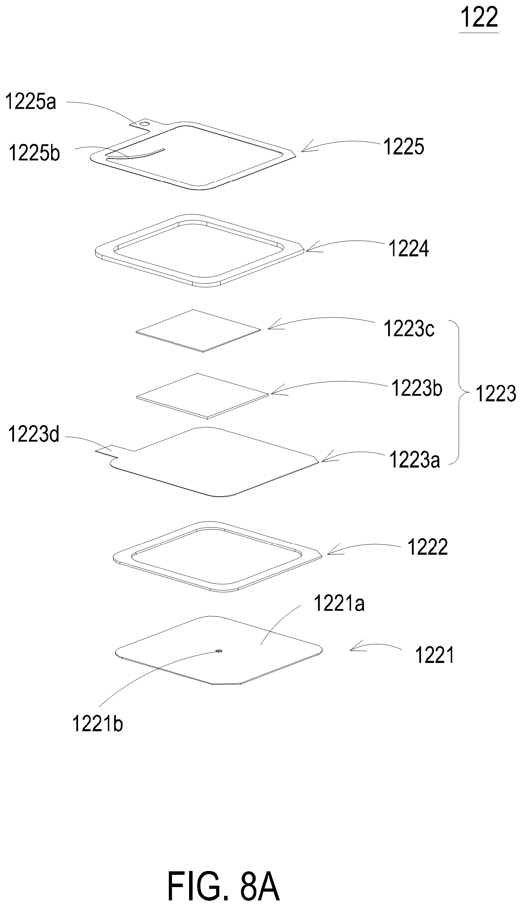

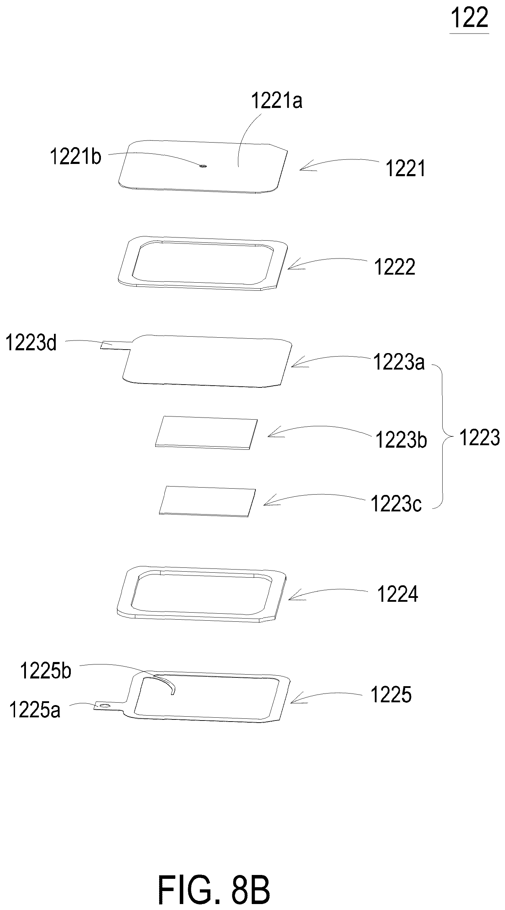

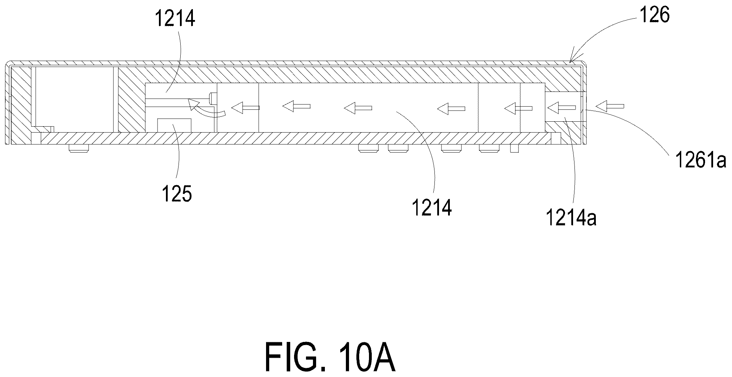

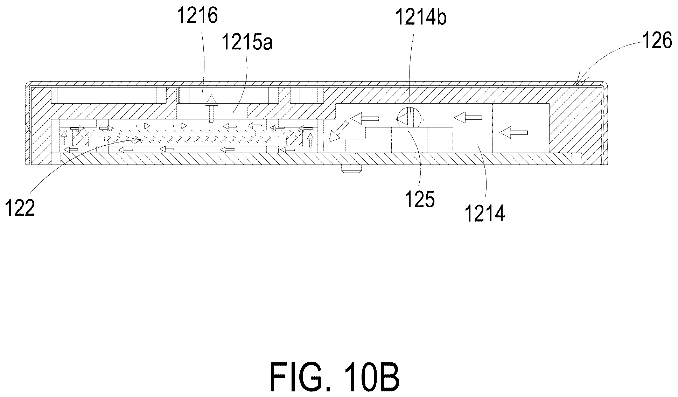

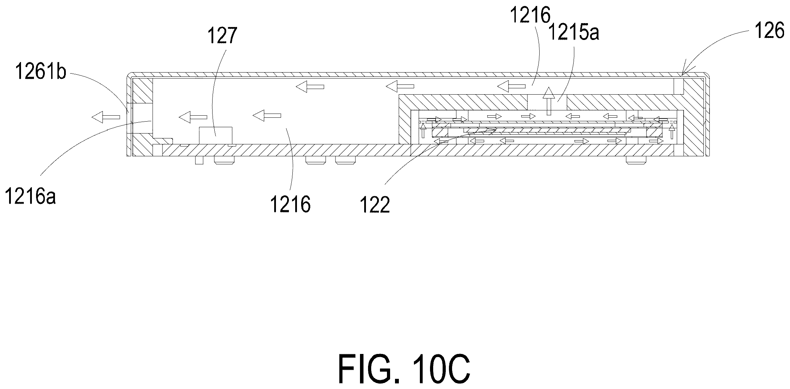

For understanding the implementation of the indoor field intelligent cleaning system in the present disclosure, followings are the detailed descriptions of the gas detection module 1. Please refer to FIG. 2A, FIG. 3A to FIG. 10. In the embodiment, the gas detection module 1 includes a controlling circuit board 11 and a gas detection main part 12. The controlling circuit board 11 includes a power conversion component 111, at least one connection interface 112, a microcontroller (MCU) 113 and a wireless communicator 114. The microcontroller (MCU) 113 and the wireless communicator 114 are arranged on the controlling circuit board 11 and electrically connected to each other. The microcontroller (MCU) 113 controls the driving signal of the gas detection main part 12 for enabling the detection. In this way, the gas detection main part 12 detects the air pollution and outputs the air quality information, and the microcontroller (MCU) 113 receives, processes and provides the detection information to the wireless communicator 114 for externally transmitting to the networked cloud computing service device 3 via IoT communication.

Please refer to FIG. 4A to FIG. 9A. The gas detection main part 12 includes a base 121, a piezoelectric actuator 122, a driving circuit board 123, a laser component 124, a particulate sensor 125, and an outer cover 126. In the embodiment, the base 121 includes a first surface 1211, a second surface 1212, a laser loading region 1213, a gas-inlet groove 1214, a gas-guiding-component loading region 1215 and a gas-outlet groove 1216. The first surface 1211 and the second surface 1212 are two surfaces opposite to each other. The laser loading region 1213 is hollowed out from the first surface 1211 toward the second surface 1212. The outer cover 126 covers the base 121 and includes a side plate 1261. The side plate 1261 has an inlet opening 1261a and an outlet opening 1261b. The gas-inlet groove 1214 is concavely formed from the second surface 1212 and disposed adjacent to the laser loading region 1213. The gas-inlet groove 1214 includes a gas-inlet 1214a and two lateral walls. The gas-inlet 1214a is in communication with an environment outside the base 121, and is spatially corresponding in position to an inlet opening 1261a of the outer cover 126. Two transparent windows 1214b are opened on the two lateral walls of the gas-inlet groove 1214 and are in communication with the laser loading region 1213. Therefore, the first surface 1211 of the base 121 is covered and attached by the outer cover 126, and the second surface 1212 is covered and attached by the driving circuit board 123, so that an inlet path is defined by the gas-inlet groove 1214. In the embodiment, the gas-guiding-component loading region 1215 is concavely formed from the second surface 1212 and in communication with the gas-inlet groove 1214. A ventilation hole 1215a penetrates a bottom surface of the gas-guiding-component loading region 1215. The gas-guiding-component loading region 1215 includes four positioning protrusions 1215b disposed at four corners of the gas-guiding-component loading region 1215, respectively. In the embodiment, the gas-outlet groove 1216 includes a gas-outlet 1216a, and the gas-outlet 1216a is spatially corresponding to the outlet opening 1261b of the outer cover 126. The gas-outlet groove 1216 includes a first section 1216b and a second section 1216c. The first section 1216b is concavely formed out from the first surface 1211 in a region spatially corresponding to a vertical projection area of the gas-guiding-component loading region 1215. The second section 1216c is hollowed out from the first surface 1211 to the second surface 1212 in a region where the first surface 1211 is extended from the vertical projection area of the gas-guiding-component loading region 1215. The first section 1216b and the second section 1216c are connected to form a stepped structure. Moreover, the first section 1216b of the gas-outlet groove 1216 is in communication with the ventilation hole 1215a of the gas-guiding-component loading region 1215, and the second section 1216c of the gas-outlet groove 1216 is in communication with the gas-outlet 1216a. In that, when first surface 1211 of the base 121 is attached and covered by the outer cover 126 and the second surface 1212 of the base 121 is attached and covered by the driving circuit board 123, the gas-outlet groove 1216 and the driving circuit board 123 collaboratively define an outlet path.

In the embodiment, the laser component 124 and the particulate sensor 125 are disposed on and electrically connected to the driving circuit board 123 and located within the base 121. In order to clearly describe and illustrate the positions of the laser component 124 and the particulate sensor 125 in the base 121, the driving circuit board 123 is intentionally omitted. The laser component 124 is accommodated in the laser loading region 1213 of the base 121, and the particulate sensor 125 is accommodated in the gas-inlet groove 1214 of the base 121 and is aligned to the laser component 124. In addition, the laser component 124 is spatially corresponding to the transparent window 1214b, so that a light beam emitted by the laser component 124 passes through the transparent window 1214b and is irradiated into the gas-inlet groove 1214. A light beam path emitted from the laser component 124 passes through the transparent window 1214b and extends in an orthogonal direction perpendicular to the gas-inlet groove 1214. In the embodiment, a projecting light beam emitted from the laser component 124 passes through the transparent window 1214b and enters the gas-inlet groove 1214 to irradiate the suspended particles contained in the gas passing through the gas-inlet groove 1214. When the suspended particles contained in the gas are irradiated and generate scattered light spots, the scattered light spots are received and calculated by the particulate sensor 125, which is in an orthogonal direction perpendicular to the gas-inlet groove 1214, to obtain the gas detection information.

In the embodiment, the piezoelectric actuator 122 is accommodated in the square-shaped gas-guiding-component loading region 1215 of the base 121. In addition, the gas-guiding-component loading region 1215 is in fluid communication with the gas-inlet groove 1214. When the piezoelectric actuator 122 is enabled, the gas in the gas-inlet groove 1214 is inhaled by the piezoelectric actuator 122, so that the gas flows into the piezoelectric actuator 122, and is transported into the gas-outlet groove 1216 through the ventilation hole 1215a of the gas-guiding-component loading region 1215. Moreover, the driving circuit board 123 covers the second surface 1212 of the base 121, and the laser component 124 is positioned and disposed on the driving circuit board 123, and is electrically connected to the driving circuit board 123. The particulate sensor 125 is also positioned and disposed on the driving circuit board 123 and electrically connected to the driving circuit board 123. In that, when the outer cover 126 covers the base 121, the inlet opening 1261a is spatially corresponding to the gas-inlet 1214a of the base 121, and the outlet opening 1261b is spatially corresponding to the gas-outlet 1216a of the base 121.

In the embodiment, the piezoelectric actuator 122 includes a gas-injection plate 1221, a chamber frame 1222, an actuator element 1223, an insulation frame 1224 and a conductive frame 1225. In the embodiment, the gas-injection plate 1221 is made by a flexible material and includes a suspension plate 1221a and a hollow aperture 1221b. The suspension plate 1221a is a sheet structure and is permitted to undergo a bending deformation. Preferably but not exclusively, the shape and the size of the suspension plate 1221a are corresponding to the inner edge of the gas-guiding-component loading region 1215, but not limited thereto. The hollow aperture 1221b passes through a center of the suspension plate 1221a, so as to allow the gas to flow therethrough. Preferably but not exclusively, in the embodiment, the shape of the suspension plate 1221a is selected from the group consisting of a square, a circle, an ellipse, a triangle and a polygon, but not limited thereto.

In the embodiment, the chamber frame 1222 is carried and stacked on the gas-injection plate 1221. In addition, the shape of the chamber frame 1222 is corresponding to the gas-injection plate 1221. The actuator element 1223 is carried and stacked on the chamber frame 1222 and collaboratively defines a resonance chamber 1226 with the chamber frame 1222 and the gas-injection plate 1221. The insulation frame 1224 is carried and stacked on the actuator element 1223 and the appearance of the insulation frame 1224 is similar to that of the chamber frame 1222. The conductive frame 1225 is carried and stacked on the insulation frame 1224, and the appearance of the conductive frame 1225 is similar to that of the insulation frame 1224. In addition, the conductive frame 1225 includes a conducting pin 1225a and a conducting electrode 1225b. The conducting pin 1225a is extended outwardly from an outer edge of the conductive frame 1225, and the conducting electrode 1225b is extended inwardly from an inner edge of the conductive frame 1225. Moreover, the actuator element 1223 further includes a piezoelectric carrying plate 1223a, an adjusting resonance plate 1223b and a piezoelectric plate 1223c. The piezoelectric carrying plate 1223a is carried and stacked on the chamber frame 1222. The adjusting resonance plate 1223b is carried and stacked on the piezoelectric carrying plate 1223a. The piezoelectric plate 1223c is carried and stacked on the adjusting resonance plate 1223b. The adjusting resonance plate 1223b and the piezoelectric plate 1223c are accommodated in the insulation frame 1224. The conducting electrode 1225b of the conductive frame 1225 is electrically connected to the piezoelectric plate 1223c. In the embodiment, the piezoelectric carrying plate 1223a and the adjusting resonance plate 1223b are made by a conductive material. The piezoelectric carrying plate 1223a includes a piezoelectric pin 1223d. The piezoelectric pin 1223d and the conducting pin 1225a are electrically connected to a driving circuit (not shown) on the driving circuit board 123, so as to receive a driving signal (which can be a driving frequency and a driving voltage). Through this structure, a circuit is formed by the piezoelectric pin 1223d, the piezoelectric carrying plate 1223a, the adjusting resonance plate 1223b, the piezoelectric plate 1223c, the conducting electrode 1225b, the conductive frame 1225 and the conducting pin 1225a for transmitting the driving signal. Moreover, the insulation frame 1224 is insulated between the conductive frame 1225 and the actuator element 1223, so as to avoid the occurrence of a short circuit, thereby transmitting the driving signal to the piezoelectric plate 1223c. After receiving the driving signal, the piezoelectric plate 1223c deforms due to the piezoelectric effect, and the piezoelectric carrying plate 1223a and the adjusting resonance plate 1223b are further driven to generate the bending deformation in the reciprocating manner.

Furthermore, in the embodiment, the adjusting resonance plate 1223b is located between the piezoelectric plate 1223c and the piezoelectric carrying plate 1223a and served as a cushion between the piezoelectric plate 1223c and the piezoelectric carrying plate 1223a. Accordingly, the vibration frequency of the piezoelectric carrying plate 1223a is adjustable. Basically, the thickness of the adjusting resonance plate 1223b is greater than the thickness of the piezoelectric carrying plate 1223a, and the vibration frequency of the actuator element 1223 can be adjusted by adjusting the thickness of the adjusting resonance plate 1223b.

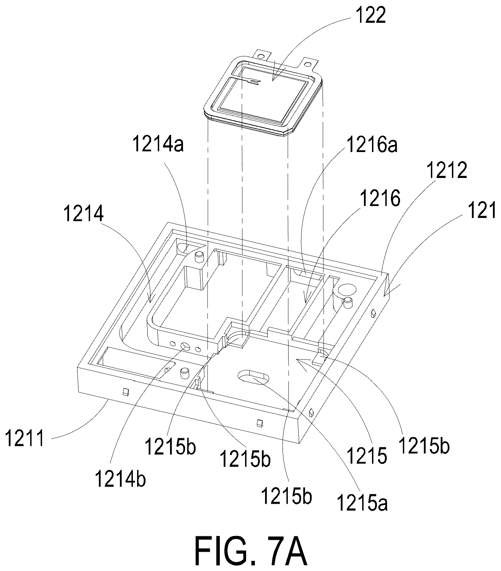

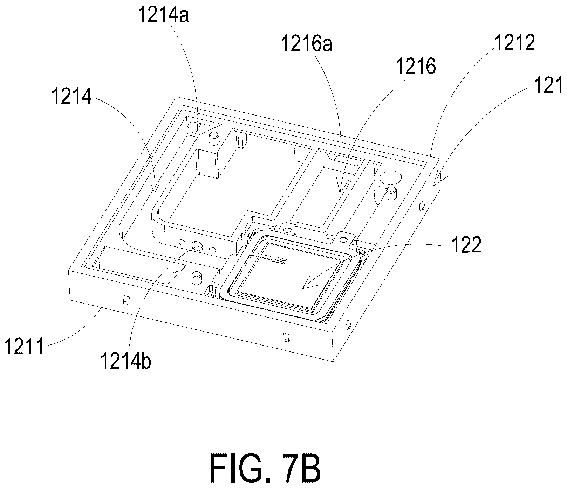

Please further refer to FIG. 7A, FIG. 7B, FIG. 8A, FIG. 8B and FIG. 9A. In the embodiment, the gas-injection plate 1221, the chamber frame 1222, the actuator element 1223, the insulation frame 1224 and the conductive frame 1225 are stacked and positioned in the gas-guiding-component loading region 1215 sequentially, so that the piezoelectric actuator 122 is supported and positioned in the gas-guiding-component loading region 1215. A clearance 1221c is defined between the suspension plate 1221a and an inner edge of the gas-guiding-component loading region 1215 for gas flowing therethrough. In the embodiment, a flowing chamber 1227 is formed between the gas-injection plate 1221 and the bottom surface of the gas-guiding-component loading region 1215. The flowing chamber 1227 is in communication with the resonance chamber 1226 among the actuator element 1223, the chamber frame 1222 and the gas-injection plate 1221 through the hollow aperture 1221b of the gas-injection plate 1221. By controlling the vibration frequency of the gas in the resonance chamber 1226 to be close to the vibration frequency of the suspension plate 1221a, the Helmholtz resonance effect is generated between the resonance chamber 1226 and the suspension plate 1221a, so as to improve the efficiency of gas transportation. When the piezoelectric plate 1223c is moved away from the bottom surface of the gas-guiding-component loading region 1215, the suspension plate 1221a of the gas-injection plate 1221 is driven to move away from the bottom surface of the gas-guiding-component loading region 1215 by the piezoelectric plate 1223c. In that, the volume of the flowing chamber 1227 is expanded rapidly, the internal pressure of the flowing chamber 1227 is decreased to form a negative pressure, and the gas outside the piezoelectric actuator 122 is inhaled through the clearance 1221c and enters the resonance chamber 1226 through the hollow aperture 1221b. Consequently, the pressure in the resonance chamber 1226 is increased to generate a pressure gradient. When the suspension plate 1221a of the gas-injection plate 1221 is driven by the piezoelectric plate 1223c to move toward the bottom surface of the gas-guiding-component loading region 1215, the gas in the resonance chamber 1226 is discharged out rapidly through the hollow aperture 1221b, and the gas in the flowing chamber 1227 is compressed, such that the converged gas is quickly and massively ejected out of the flowing chamber 1227 under the condition close to an ideal gas state of the Bernoulli's law, and transported to the ventilation hole 1215a of the gas-guiding-component loading region 1215.

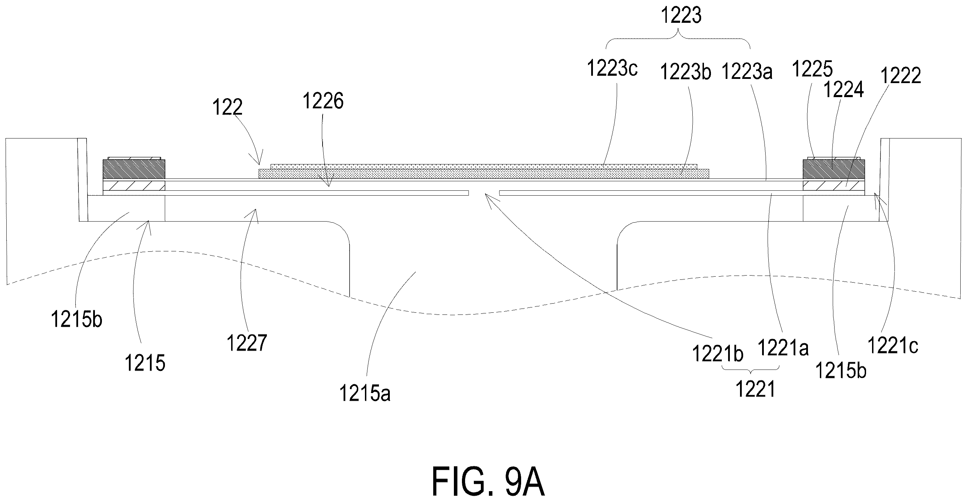

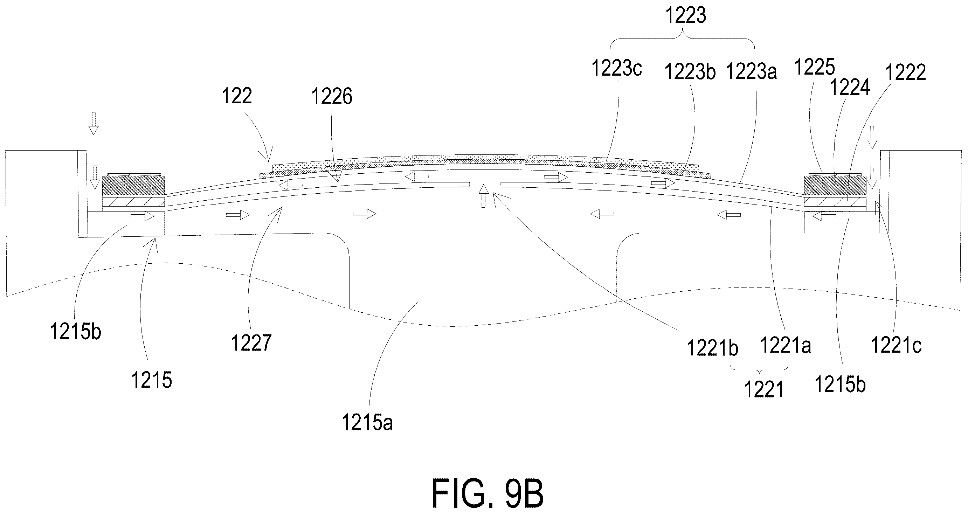

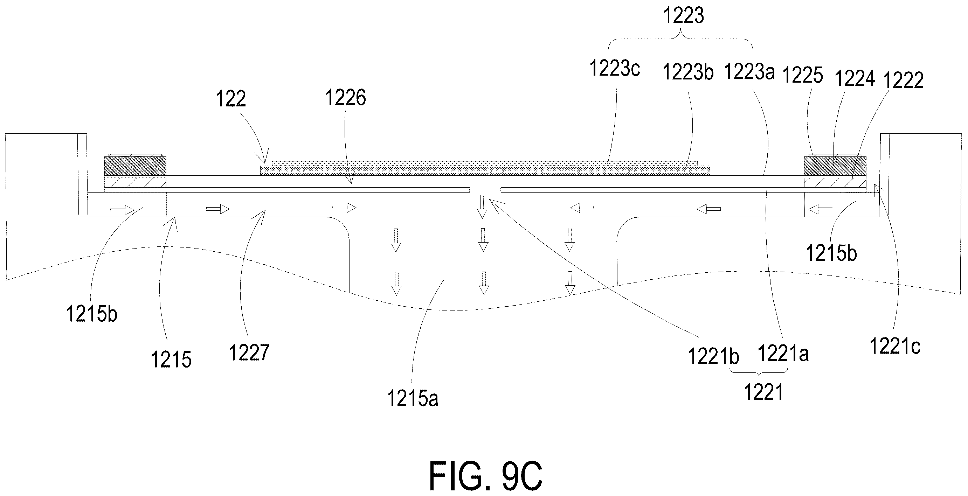

By repeating the above operation steps shown in FIG. 9B and FIG. 9C, the piezoelectric plate 1223c is driven to generate the bending deformation in a reciprocating manner. According to the principle of inertia, since the gas pressure inside the resonance chamber 1226 is lower than the equilibrium gas pressure after the converged gas is ejected out, the gas is introduced into the resonance chamber 1226 again. Moreover, the vibration frequency of the gas in the resonance chamber 1226 is controlled to be close to the vibration frequency of the piezoelectric plate 1223c, so as to generate the Helmholtz resonance effect to achieve the gas transportation at high speed and in large quantities. The gas is inhaled through the gas-inlet 1214a on the outer cover 126, flows into the gas-inlet groove 1214 of the base 121 through the gas-inlet 1214a, and is transported to the position of the particulate sensor 125. The piezoelectric actuator 122 is enabled continuously to inhale the gas into the inlet path, and facilitate the gas outside the gas detection module to be introduced rapidly, flow stably, and transported above the particulate sensor 125. At this time, a projecting light beam emitted from the laser component 124 passes through the transparent window 1214b to irritate the suspended particles contained in the gas flowing above the particulate sensor 125 in the gas-inlet groove 1214. When the suspended particles contained in the gas are irradiated and generate scattered light spots, the scattered light spots are received and calculated by the particulate sensor 125 for obtaining related information about the sizes and the concentration of the suspended particles contained in the gas. Moreover, the gas above the particulate sensor 125 is continuously driven and transported by the piezoelectric actuator 122 to flow into the ventilation hole 1215a of the gas-guiding-component loading region 1215, and is transported to the gas-outlet groove 1216. At last, after the gas flows into the gas outlet groove 1216, the gas is continuously transported into the gas-outlet groove 1216 by the piezoelectric actuator 122, so that the gas in the gas-outlet groove 1216 is pushed to discharge through the gas-outlet 1216a and the outlet opening 1261b.

The gas detection module 1 of the present disclosure not only can detect the particulate matters in the gas, but also can detect the gas characteristics of the introduced gas, for example, to determine whether the gas is formaldehyde, ammonia, carbon monoxide, carbon dioxide, oxygen, ozone, or the like. Therefore, in some embodiments, the gas detection module 1 of the present disclosure further includes a gas sensor 127 positioned and disposed on the driving circuit board 123, electrically connected to the driving circuit board 123, and accommodated in the gas-outlet groove 1216, so as to detect the gas characteristics of the introduced gas. Preferably but not exclusively, in an embodiment, the gas sensor 127 includes a volatile-organic-compound sensor for detecting the information of carbon dioxide (CO₂) or volatile organic compounds (TVOC). Preferably but not exclusively, in an embodiment, the gas sensor 127 includes a formaldehyde sensor for detecting the information of formaldehyde (HCHO) gas. Preferably but not exclusively, in an embodiment, the gas sensor 127 includes a bacteria sensor for detecting the information of bacteria or fungi in the gas. Preferably but not exclusively, in an embodiment, the gas sensor 127 includes a virus sensor for detecting the information of virus in the gas. Preferably but not exclusively, the gas sensor 127 is a temperature and humidity sensor for detecting the temperature and humidity information of gas.

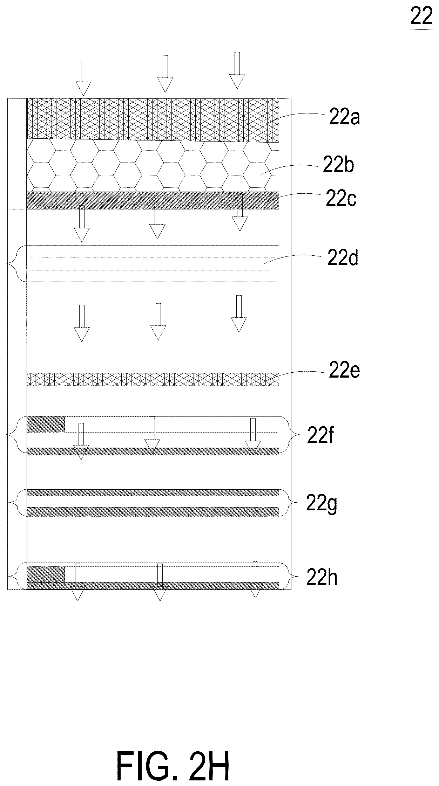

Please refer to FIG. 2H. The air guiding fan 21 of the air pollution purification apparatus 2 is controlled and enabled to guide the air pollution to pass through the filter element 22 for filtration. In the embodiment, the filter element 22 can be but not limited to be a filter 22a. The filter 22a includes a MERV (Minimum Efficiency Reporting Value) 8 filter screen or above or a high efficiency particulate air (HEPA) filter screen which is configured to absorb the chemical smoke, the bacteria, the dust particles and the pollen contained in the air pollution for achieving the effect of filtration and purification. Notably, the HEPA filter screen in the present disclosure is a HEPA 10 filter screen or above with a dust containing capacity larger than 12000 mg. In another embodiment, the filter 22a is a more efficient ULPA (Ultra Low Penetration Air) 14 filter screen for further improving the filtration efficiency and meeting higher cleanness requirements. The filter element 22 is further combined with a material for providing sterilization effect in physical or chemical means by passing therethrough the air pollution, and the airflow of the air guiding fan 21 flows in the path indicated by the arrows. The filter element 22 is combined with a decomposition layer through coating for sterilizing in chemical means by passing therethrough the air pollution. Preferably but not exclusively, the decomposition layer includes an activated carbon 22b configured to remove organic and inorganic substances in the air pollution, and remove colored and odorous substances. Notably, the activated carbon 22b in the present disclosure has an absorptive capacity of formaldehyde larger than 1500 mg. Furthermore, in some embodiments, the filter element 22 is combined with a light irradiation element for sterilizing in chemical means by passing therethrough the air pollution. Preferably but not exclusively, the light irradiation element is a photo-catalyst unit including a photo catalyst 22c and an ultraviolet lamp 22d for further improving the removal efficiency of pollutants and allergens in the air. When the photo catalyst 22d is irradiated by the ultraviolet lamp 22d, the light energy is converted into the electrical energy, thereby decomposing harmful substances and disinfects bacteria contained in the air pollution, so as to achieve the effects of filtration and purification. Notably, the power of the ultraviolet lamp 22d is larger than 120 mW. Preferably but not exclusively, the light irradiation element is a photo-plasma unit including a nanometer irradiation tube 22e. When the introduced air pollution is irradiated by the nanometer irradiation tube 22e, the oxygen molecules and water molecules contained in the air pollution are decomposed into high oxidizing photo-plasma, and an ion flow capable of destroying organic molecules is generated. In that, volatile formaldehyde, volatile toluene and volatile organic compounds (VOC) contained in the air pollution are decomposed into water and carbon dioxide, for further improving the removal efficiency of pollutants and allergens in the air, thereby achieving the effects of filtration and purification. Moreover, in some embodiments, the filter element 22 is combined with a decomposition unit for sterilizing in chemical means by passing therethrough the air pollution. Preferably but not exclusively, the decomposition unit is a negative ion unit 22f which makes the suspended particles carrying positive charges in the air pollution to adhere to negative charges for further improving the removal efficiency of pollutants and allergens in the air, thereby achieving the effects of filtration and purification. Preferably but not exclusively, the decomposition unit is a plasma ion unit 22g. The oxygen molecules and water molecules contained in the air pollution are decomposed into positive hydrogen ions (H⁺) and negative oxygen ions (O²⁻) by the plasma ion. The substances attached with water around the ions are adhered on the surfaces of viruses and bacteria and converted into OH radicals with extremely strong oxidizing power, thereby removing hydrogen (H) from the protein on the surfaces of viruses and bacteria, and thus decomposing (oxidizing) the protein. Accordingly, the pollutants, allergens and microorganism in the air can be decomposed and eliminated to improve the air cleanness and thus filter the introduced air pollution, thereby achieving the effects of filtration and purification. Preferably but not exclusively, the decomposition unit is an electrostatic filtration unit 22h, which uses electrostatic force to capture and remove the suspended particles (such as dust, pollen, bacteria and other pollutants) in the air.

In conclusion, the present disclosure provides an indoor field intelligent cleaning system. In the system, through the gas detection module continuously monitoring the air quality data, including temperature, humidity, concentration of carbon dioxide, PM2.5, for automatically enabling the air pollution purification apparatus, and simultaneously, through cooperating with the AI smart calculation platform of the networked cloud computing service device with functions of AI smart control, intelligent energy management and malfunction diagnosis, the air condition of the indoor field can rapidly be adjusted according to the real time variation of environment, thereby optimizing the systematic energy efficiency and maintaining the best air quality. Accordingly, a continuously clean and healthy air environment of the indoor field can be created, and the impact of harmful pollutants in the air on human health also can be reduced. The present disclosure includes the industrial applicability and the inventive steps.

## Claims

1. An indoor field intelligent cleaning system, **characterized by** comprising:
a plurality of gas detection modules (1) arranged in an indoor field (A) and an outdoor field (B) for detecting an air pollution and outputting an air quality data via Internet of Things (IoT) communication;
at least one air pollution purification apparatus (2) disposed in the indoor field (A) and comprising at least one gas detection module (1), at least one air guiding fan (21), at least one filter element (22) and at least one host driving controller (23), wherein the at least one gas detection module (1) is electrically connected to the at least one host driving controller (23) and receives a control instruction via Internet of Things (IoT) communication for enabling the at least one air guiding fan (21) to circulate and purify the air pollution in the indoor field (A) so as to reach a cleanness of cleanroom class with a level of air pollution close to zero; and
at least one networked cloud computing service device (3) receiving the air quality data outputted by the plurality of gas detection modules (1) via Internet of Things (IoT) communication, and performing real-time monitoring and analysis based on the collected air quality data;
wherein the at least one networked cloud computing service device (3) collects and monitors the air quality data in real time and intelligently issues a control instruction to the at least one gas detection module (1), so that the host driving controller (23) is regulated to control an activation operation of the at least one air guiding fan (21), and dynamically adjust an operating frequency and an output air volume of the at least one air guiding fan (21) so as to circulate and purify the air pollution in the indoor field (A), thereby reaching the cleanness of cleanroom class with the level of air pollution close to zero.

2. The indoor field intelligent cleaning system according to claim 1, wherein the air quality data comprises particle matters, a concentration of carbon dioxide (CO₂), temperature, and humidity.

3. The indoor field intelligent cleaning system according to claim 1, wherein the at least one networked cloud computing service device (3) comprises an AI smart calculation platform (35), and the AI smart calculation platform (35) comprises a function of AI smart control for performing a calculation based on the air quality data and automatically adjusting parameters of air volume and purification mode through a preset calculation, so as to precisely control an operation of the at least one air pollution purification apparatus (2) according to the air quality data detected in the indoor field (A) in real time, thereby optimizing systematic energy efficiency and maintaining best air quality, and the AI smart calculation platform (3) further comprises a function of intelligent energy management for dynamically adjusting a usage of energy according to an environmental condition of the indoor field (A) and an operation of the at least one air pollution purification apparatus (2).

4. The indoor field intelligent cleaning system according to claim 1, wherein the indoor field intelligent cleaning system further comprises at least one central control computer apparatus (4) and at least one airtight window (2g), the central control computer apparatus (4) receives the control instruction issued by the at least one networked cloud computing service device (3) via Internet of Things (IoT) communication, transmits the control instruction to the at least one gas detection module (1) of the at least one air pollution purification apparatus (2) for enabling the at least one air guiding fan (21), the airtight window (2g) includes a filter window (2h), when the at least one gas detection module (1) detects that the concentration of carbon dioxide (CO₂) of the air quality data of the indoor field (A) is too high, the airtight window (2g) can be opened to allow gas exchange between the indoor field (A) and the outdoor field (B), wherein the air pollution from the outdoor field (B) can be filtered through the filter window (2h) to prevent it from entering the indoor field (A), and the at least one air pollution purification apparatus (2) continuously monitors and automatically adjusts to purify the air pollution in the indoor field (A) close to zero, thereby effectively saving energy, minimizing energy consumption and maintaining a best air quality of the indoor field (A).

5. The indoor field intelligent cleaning system according to claim 4, wherein the filter window (2h) comprises an electrostatic filter or a filter woven from nanofibers, so as to prevent the air pollution of the outdoor field (B) from entering the indoor filed (A).

6. The indoor field intelligent cleaning system according to claim 1, wherein the indoor field intelligent cleaning system further comprises an airtight window (2g) including a gas exchange channel (2i), wherein the gas exchange channel (2i) is fluidly communicated with the outdoor field (B) and an inlet of the at least one air pollution purification apparatus (2), so that the air in the outdoor field (B) is introduced by the at least one air guiding fan (21), enters the indoor field (A) through the gas exchange channel (2i) and the at least one filter element (22) for gas exchange, so as to maintain the balance of carbon dioxide (CO₂) of the air quality data between the indoor field (A) and the outdoor field (B).

7. The indoor field intelligent cleaning system according to claim 1, wherein the gas detection module (1) comprises a gas detection main part (12) and a controlling circuit board (11), the gas detection main part (12) detects humidity, the temperature and the air pollution to generate the air quality data, the controlling circuit board (11) collects, calculates, analyzes and outputs the air quality data to form a serial communication (IIC) signal for input, and the networked cloud computing service device (3) receives and analyzes the air quality data in real time to output a Universal Asynchronous Transceiver and Transceiver (UART) signal and a General Purpose Input and Output (GP I/O) signal for the host driving controller (23).

8. The indoor field intelligent cleaning system according to claim 7, wherein the controlling circuit board (11) comprises:
at least one connection interface (112);
a power conversion component (111), providing DC voltage division modulation to output a required DC voltage, wherein the required DC voltage is transmitted through the at least one connection interface (112) to the gas detection main part (12) for actuation operation and to the host driving controller (23) for actuation operation;
a microcontroller (MCU) (113), connected to the gas detection main part (12) through the at least one connection interface (112) to form the serial communication (IIC) signal for input, so that the air quality data is calculated and analyzed, and connected through the at least one connection interface (112) to output the Universal Asynchronous Transceiver and Transceiver (UART) signal and the General Purpose Input and Output (GP I/O) signal for regulation; and
a wireless communicator (114), receiving the air quality data and transmitting to the networked cloud computing service device (3) through external wireless communication, wherein the networked cloud computing service device (3) collects, analyzes and monitors the air quality data in real time and intelligently selects the control instruction to the wireless communicator (114), and then transmits the control instruction to the microcontroller (MCU) (113) to output the Universal Asynchronous Transceiver and Transceiver (UART) signal and the General Purpose Input and Output (GP I/O) signal for regulation of the host driving controller (23), so that the host driving controller (23) is regulated to control the activation operation of the at least one air guiding fan (21), and dynamically adjust the operating frequency and the output air volume of the at least one air guiding fan (21).

9. The indoor field intelligent cleaning system according to claim 8, further comprising a wired communication port (13), wherein the wired communication port (13) is electrically connected to the controlling circuit board (11) through the connection interface (112) for external connection to a wired communication transmission, wherein the air quality data is received and transmitted to the networked cloud computing service device (3) through external wireless communication, the networked cloud computing service device (2) collects, analyzes and monitors the air quality data in real time and intelligently selects a control command, and the control command is received through the wired communication port (13) and transmitted to the microcontroller (MCU) (113) to output the Universal Asynchronous Transceiver and Transceiver (UART) signal and the General Purpose Input and Output (GP I/O) signal for regulation of the host driving controller (23), so that the host driving controller (23) is regulated to control the activation operation of the at least one air guiding fan (21), and dynamically adjust the operating frequency and the output air volume of the at least one air guiding fan (21).

10. The indoor field intelligent cleaning system according to claim 1, wherein the at least one air pollution purification apparatus (2) comprises a gas exchange device (2a), a purifier (2b), a fan filter unit (FFU) (2c), an exhaust fan (2d), a heating and cooling air conditioner (2e), and a humidity controller (2f); wherein the gas exchange device (2a) comprises a fresh air exchanging system, an energy recovery ventilation system, or a heating, ventilation and air conditioning (HVAC) system; wherein the heating and cooling air conditioner (2e) comprises an air cooling device, an air heat generating device or an air cooling and heating device.

11. The indoor field intelligent cleaning system according to claim 1, wherein the at least one filter element (22) is a MERV (Minimum Efficiency Reporting Value) 8 filter screen or a HEPA (High Efficiency Particulate Air) 10 filter screen or above with a dust containing capacity larger than 12000 mg.

12. The indoor field intelligent cleaning system according to claim 1, wherein the at least one filter element (22) is a ULPA (Ultra Low Penetration Air) 14 filter screen or above, and the at least one filter element (22) is combined with a decomposition layer through coating for sterilizing in chemical means by passing therethrough the air pollution, and the decomposition layer comprises an activated carbon (22b) having an absorptive capacity of formaldehyde larger than 1500 mg.

13. The indoor field intelligent cleaning system according to claim 1, wherein the at least one filter element (22) is combined with a decomposition unit for sterilizing in chemical means by passing therethrough the air pollution.

14. The indoor field intelligent cleaning system according to claim 1, wherein the IoT communication comprises a wireless communication or a wired communication, the wireless communication is used for the at least one networked cloud computing service device (3) to communicate wirelessly, wherein the wireless communication is one selected from the group consisting of a Wi-Fi communication, a Bluetooth communication, a radio frequency identification communication and a near field communication, and the wired communication is used for the at least one networked cloud computing service device (3) to communicate in a wired manner.

15. The indoor field intelligent cleaning system according to claim 4, wherein the at least one central control computer apparatus (4) is equipped with a function of edge computing for calculating and analyzing the air quality data received from the at least one gas detection module (1) in each of the at least one air pollution purification apparatus (2), generating the control instruction based on an analysis result and directly issuing the control instruction to the at least one gas detection module (1) in each of the at least one air pollution purification apparatus (2) via Internet of Things (IoT) communication so as to enable the at least one air guiding fan (21), thereby achieving an automatic control and an optimization of the at least one air pollution purification apparatus (2).
